(19) Europäisches Patentamt / European Patent Office / Office européen des brevets

(11) **EP 1 941 279 B1**

(12) **FASCICULE DE BREVET EUROPEEN**

(45) Date de publication et mention de la délivrance du brevet:
**22.10.2014 Bulletin 2014/43**

(51) Int Cl.:
*G01N 33/58* $^{(2006.01)}$   *A61K 49/00* $^{(2006.01)}$

(21) Numéro de dépôt: **06807674.4**

(22) Date de dépôt: **30.10.2006**

(86) Numéro de dépôt international:
**PCT/EP2006/067950**

(87) Numéro de publication internationale:
**WO 2007/048856 (03.05.2007 Gazette 2007/18)**

(54) **NANOPARTICULES À LUMINESCENCE PERSISTANTE POUR LEUR UTILISATION EN TANT QU'AGENT DE DIAGNOSTIC DESTINÉ À L'IMAGERIE OPTIQUE IN VIVO**

IN FORM EINES DIAGNOSEMITTELS ZUR OPTISCHEN ABBILDUNG IN VIVO VERWENDETE NANOPARTIKEL MIT ANHALTENDER LUMINESZENZ

PERSISTENT LUMINESCENCE NANOPARTICLES USED IN THE FORM OF A DIAGNOSIS AGENT FOR IN VIVO OPTICAL IMAGING

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.10.2005 FR 0511113**

(43) Date de publication de la demande:
**09.07.2008 Bulletin 2008/28**

(73) Titulaires:
  • **CENTRE NATIONAL DE LA RECHERCHE SCIENTIFIQUE (CNRS)**
    **75016 Paris (FR)**
  • **INSTITUT NATIONAL DE LA SANTE ET DE LA RECHERCHE MEDICALE (INSERM)**
    **75013 Paris (FR)**

(72) Inventeurs:
  • **SCHERMAN, Daniel**
    **F-75012 Paris (FR)**
  • **BESSODES, Michel**
    **F-94800 Villejuif (FR)**
  • **CHANEAC, Corinne**
    **F-93170 Bagnolet (FR)**
  • **GOURIER, Didier, Louis**
    **F-75013 Paris (FR)**
  • **JOLIVET, Jean-Pierre**
    **F-91640 Vaugrigneuse (FR)**
  • **LE MASNE DE CHERMONT, Quentin**
    **F-75005 Paris (FR)**
  • **MAITREJEAN, Serge**
    **F-75020 Paris (FR)**
  • **PELLE, Fabienne, Julie**
    **F-92700 Colombes (FR)**

(74) Mandataire: **Regimbeau**
    **20, rue de Chazelles**
    **75847 Paris Cedex 17 (FR)**

(56) Documents cités:
    **US-A1- 2003 059 635     US-A1- 2003 180 780**
    **US-A1- 2004 014 060     US-A1- 2005 136 258**
    **US-B1- 6 537 829**

  • **MEHTA ADOSH ET AL: "Size-correlated spectroscopy and imaging of rare-earth-doped nanocrystals." APPLIED OPTICS. 20 APR 2003, vol. 42, no. 12, 20 avril 2003 (2003-04-20), pages 2132-2139, XP002401761 ISSN: 0003-6935**

EP 1 941 279 B1

## Description

La présente invention a pour objet l'utilisation de nanoparticules à luminescence persistante, le cas échéant fonctionnalisées, en tant qu'agent de diagnostic destiné à l'imagerie optique *in vivo.* Ces nanoparticules sont de préférence constituées par un composé choisi dans le groupe constitué par (1) les silicates, les aluminates, les aluminosilicates, les germanates, les titanates, les oxysulfures, les phosphates et les vanadates, de tels composés comprenant au moins un oxyde de métal, (2) les sulfures comprenant au moins un ion métallique choisi parmi le zinc, le strontium et le calcium, et (3) les oxydes de métaux, ledit composé étant dopé avec au moins un ion terre rare, et éventuellement avec au moins un ion d'un métal de transition. De préférence, l'agent de diagnostic est destiné à l'imagerie de la vascularisation de l'organisme ou des organes réticulo-endothéliaux (foie, rate). L'invention a encore pour objet un procédé et un kit de détection ou de quantification *in vitro* d'une substance d'intérêt biologique ou chimique dans un échantillon, par mise en oeuvre desdites nanoparticules préalablement fonctionnalisées.

Le développement rapide de nombreuses techniques d'imagerie durant les dernières décennies (IRM, écho-doppler, scanner, Tomographie par émission de positons...) répond à un besoin croissant de la part des biologistes et des médecins. De la simplification des travaux expérimentaux à une détection précoce de maladies, une dynamique s'est créée autour des recherches en imagerie. Chaque technique possède avantages et inconvénients, rendant ainsi l'ensemble des imageries complémentaires.

L'imagerie optique, utilisant les photons comme source d'information, est essentiellement utilisée pour des études *in vitro.* Il s'agit d'un domaine en pleine expansion avec des retombées directes en pharmacologie, dans le développement d'outils d'aide au diagnostic et de recherches en biologie moléculaire et cellulaire. Les autres techniques d'imagerie trouvent la majorité de leurs applications en imagerie *in vivo,* que ce soit chez le petit animal ou chez l'homme. Une place toujours croissante est accordée à ces techniques dans les études de biodistribution en biologie. Il est en effet possible de réaliser un suivi dynamique et longitudinal sur chaque animal en diminuant de façon notable le sacrifice d'animaux. Cependant le coût élevé et un certain nombre de barrières technologiques rendent difficile leur utilisation quotidienne.

Avec le développement de nouveaux capteurs optiques plus sensibles (sensibilité de l'ordre du photon) et de nouvelles sondes performantes, l'imagerie optique commence à se pencher sur la problématique des études *in vivo,* et ce avec des coûts plus faibles.

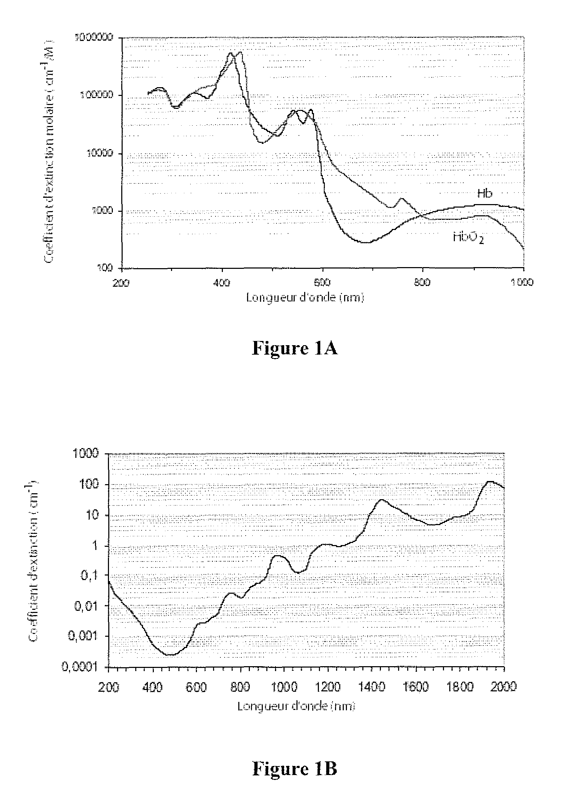

L'imagerie optique utilise essentiellement des sondes fluorescentes, qu'elles soient moléculaires organiques (rhodamine, bromure d'éthidium...), biologiques (protéines de type GFP et analogues) ou inorganiques (Quantum Dots). Toutefois, l'autofluorescence des tissus et de l'ensemble des composants organiques, surtout en excitation UV rend l'utilisation de la fluorescence difficile. Le problème se complexifie encore dans les études *in vivo* à cause de la diffusion des photons par les tissus, ce qui altère la forme et la taille de la zone observée. En effet, deux phénomènes physiques bien connus - l'absorption et la diffusion - limitent la propagation d'ondes électromagnétiques dans un milieu, qu'il soit biologique ou non. De plus, l'atténuation de la lumière dans les tissus rend l'observation des tissus profonds difficile, si la propriété d'émission de la sonde ne se trouve pas dans la zone de transparence des tissus (longueur d'onde allant de 650 nm à l'infrarouge). Une illustration en est donnée aux Figures 1A (spectre d'absorption de l'hémoglobine) et 1B (spectre d'absorption de l'eau). Entre 400 et 600 nm, l'absorption de l'ensemble des constituants du milieu biologique est très importante pour une imagerie des tissus profonds. Au-delà de 1300 nm, les ondes électromagnétiques sont absorbées sous forme d'énergie thermique par l'ensemble des molécules (surtout par les molécules d'eau).

De surcroît, dans de nombreux cas, les composés fluorescents sont phototoxiques. En effet, quand un fluorophore (endogène ou exogène) est excité, il peut y avoir création d'un état triplet. Cet état est très réactif chimiquement et peut endommager les cellules vivantes. Un des mécanismes entraînant le plus de dommages correspond à la génération d'un oxygène singulet, donnant lieu à la chaîne du stress oxydatif.

Tous ces facteurs limitent ainsi fortement le suivi des molécules d'intérêt sur l'animal vivant. Pour ces raisons, la biodistribution de composés fluorescents n'est réellement analysable que *"ex vivo",* c'est-à-dire en sacrifiant les animaux pour prélever les organes, extraire le fluorophore et évaluer la quantité par rapport à l'étalonnage correspondant à chaque organe. Mais, dans ce cas encore, des difficultés importantes proviennent du fait de la photo-désactivation du chromophore organique après quelques cycles d'excitation, qui altère rapidement sa fluorescence.

Ainsi, bien que les progrès aient été spectaculaires au niveau de la technologie des caméras et autres détecteurs, il reste donc encore beaucoup à faire pour développer les sondes utilisables *in vivo* pour l'observation optique.

Il est nécessaire de faire la distinction entre les différents mécanismes physiques qui font qu'une molécule ou une nanoparticule peut émettre de la lumière après excitation.

*Luminescence* est le terme générique pour caractériser les substances qui vont restituer sous forme de photons d'origine non thermique une partie de l'énergie absorbée au cours d'une excitation non thermique. L'excitation de ces composées se fait par apport d'énergie qui peut prendre de nombreuses formes. On peut citer, sans être exhaustif, l'excitation par longueurs d'ondes de l'ultraviolet (UV), du visible ou de l'infrarouge (IR), par des rayonnements X, des réactions chimiques (chimiluminescence), des réactions enzymatiques (bioluminescence), des excitations électriques (électroluminescence) ou encore des excitations mécaniques (triboluminescence).

Phénomènes physiques donnant lieu à luminescence

Il est pertinent de séparer les phénomènes de luminescence selon la nature de la substance émettant des photons, qu'il s'agisse d'une molécule ou d'un matériau.

Pour une molécule :

La luminescence est une désactivation d'une molécule excitée vers un état énergétique moins élevé. Ce phénomène se différencie classiquement en deux types selon la durée séparant l'absorption de la ré-émission. Si cette durée est courte, on parle de *fluorescence ;* si elle est plus longue, on parle de *phosphorescence.*

Le mécanisme de la fluorescence est le suivant : les photons d'une source lumineuse peuvent être absorbés par la molécule, la faisant passer de l'état fondamental à un état excité. Cette absorption de lumière se fait de façon rapide (environ $10^{-15}$ s). La relaxation à l'état singulet excité de plus faible énergie, appelée conversion interne, se produit par échange thermique avec le milieu en environ $10^{-11}$ s. Ensuite, chaque molécule peut perdre son énergie selon plusieurs voies. Il peut se désexciter soit de façon radiative en émettant un photon - c'est la *fluorescence* qui se produit en un temps de l'ordre de la nanoseconde - soit de façon non radiative en transformant cette énergie en énergie de rotation ou de vibration.

Toutefois un autre phénomène peut se produire. Il s'agit du transfert de l'énergie dans un état excité triplet par croisement inter-système. Pour des raisons quantiques, la désexcitation d'un état triplet à un état singulet (l'état fondamental) est impossible. L'électron reste donc bloqué dans cette position pendant un temps relativement long (de quelques millisecondes à quelques secondes), avant que des modifications de son environnement lui permettent une désexcitation radiative. C'est la *phosphorescence.* Il est à noter que, à la différence de la fluorescence, la durée de phosphorescence est grandement affectée par la température. En général, les temps d'émission sont plus longs à basse température qu'à température ambiante.

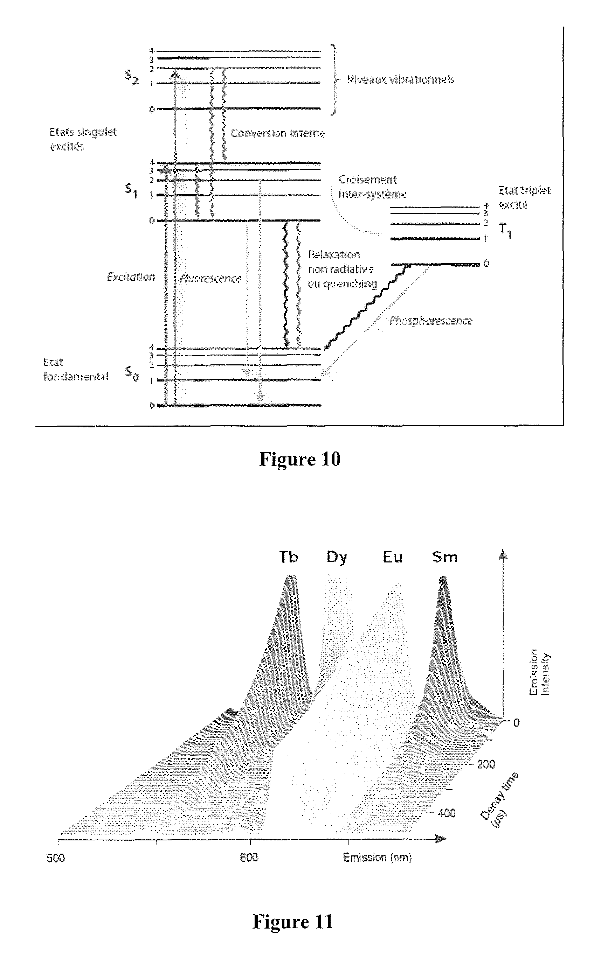

Ces différents scénarios sont résumés dans le diagramme de radiative (Fig. 10). Ce diagramme illustre les différentes transitions électroniques ainsi que les différents phénomènes de désexcitation.

Un autre cas un peu particulier existe. Il se situe, en terme de durée d'émission, entre la fluorescence et la phosphorescence. Il s'agit de la *fluorescence retardée* (en anglais : long lived fluorescence). Il est en effet possible à partir de l'état $T_1$ de retourner à l'état $S_1$. Pour cela, la molécule doit gagner de l'énergie soit par collision, soit par absorption d'un autre photon, soit par transfert d'énergie entre deux états $T_1$. La fluorescence observée apparaît alors plus tard dans le temps après de phénomène d'absorption, tout en ayant le même spectre d'émission que celui observé en fluorescence spontanée.

Un autre cas où on parle de fluorescence retardée est celui des chélates de lanthanides qui peuvent être décrit comme suivant : un motif chromophore organique collectant l'énergie d'excitation est greffé sur un ligand complexant un cation lanthanide inorganique (le plus souvent europium, terbium ou ruthénium). Dans certains cas, le chélatant sert directement de motif chromophore captant l'excitation. Le temps de transfert d'énergie ainsi que des considérations quantiques sur les terres rares font que la durée d'émission des ces complexes peut atteindre plusieurs ms (Fig. 11)

Pour un matériau :

Les types de matériaux pouvant être utilisées comme sondes optiques sont de façon non exhaustives:

- les nanocristaux semi-conducteurs luminescents (« quantum dots »),
- les matériaux dopés avec des ions terre rare ou métaux de transitions,
- les nanoparticules de silice ou de polymère incorporant ou fonctionnalisées par les radiative préalablement cités (radiative organiques, complexes de lanthanides, « quantum dots »).

*Quantum dots*

Les « quantum dots » (qdots) sont des nanocristaux inorganiques de taille nanométrique. Ils sont composés d'un noyau semi-conducteur inorganique (CdS, CdSe, ZnO, InP, InAs,....) responsable de l'émission lumineuse, et sont en général recouverts d'une coquille inorganique (ZnS) augmentant le rendement quantique et limitant le photo-blanchiment. La taille du cristal inorganique varie généralement entre 2 et 8 nm. Il est enfin nécessaire de les fonctionnaliser par des molécules organiques pour y greffer des biomolécules et les utiliser pour des applications biologiques.

La luminescence observée pour les qdots est due à la recombinaison d'une paire électron-trou créée lors de l'excitation lumineuse dans le coeur nanocristal semi-conducteur. Cette recombinaison se fait de façon relativement longue (environ 20 à 30 ns) par rapport au radiative classique. La durée est toutefois suffisamment courte pour que le terme fluorescence qualifie la luminescence des qdots.

*Matériaux dopés*

Pour leur conférer des propriétés optiques, ces matériaux sont dopés avec des ions absorbeurs et émetteurs, majoritairement des cations lanthanides ou des ions de transitions. Pour les matériaux dopés avec des ions terres rares, les propriétés optiques très similaires à celles décrites précédemment pour les complexes de lanthanides avec une durée de luminescence de l'ordre de la ms. Pour les matériaux dopés avec des ions de transitions ou autres, la luminescence obtenue est due à une émission de fluorescence des dopants.

*Nanoparticule de silice ou de polymère incorporant ou fonctionnalisées par les fluorophores préalablement cités.*

Les nanoparticules de polymère sont constituées d'une matrice de polymère organique telle que le polystyrène, le poly-méthylmétacrylate (PMMA), des dextrans, etc. Les fluorophores peuvent être piégés dans la matrice au cours de sa synthèse, ou postérieurement par gonflement du polymère et diffusion des fluorophores à l'intérieur. Une autre méthode consiste à polymériser directement un monomère marqué par un fluorophore organique.

Les nanoparticules de silice sont obtenues par la synthèse de radiative modifiée. Cette synthèse consiste en l'hydrolyse radiative en présence du radiative à encapsuler dans la matrice de silice, en créant une micro-émulsion en présence d'un surfactant, afin de contrôler la taille des particules obtenues (la taille peut aller de 20 nm à 1 $\mu$m) et la charge en radiative

Par des méthodes de fonctionnalisation, il est également possible de greffer sur ce type de nanoparticules (comme sur toutes nanoparticules ou matériau) l'ensemble des fluorophores citées précédemment.

Phénomène d'upconversion.

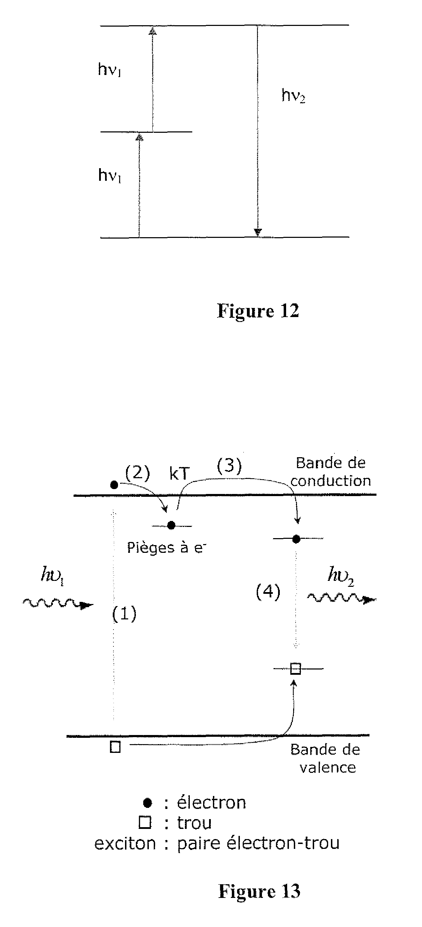

L'excitation photonique à une certaine longueur d'onde (par exemple dans l'infrarouge) qui est suivi d'une luminescence à une longueur d'onde plus courte (par exemple dans le visible) est appelée « upconversion ». Il s'agit d'un processus assez inhabituel puisque des photons de faible énergie sont « transformés » en photons d'énergie supérieure (Fig. 12). Au moins deux photons IR sont nécessaires pour générer un photon dans le visible. Le phénomène d'upconversion ne peut arriver que dans des matériaux pour lesquels la relaxation multi phonon n'est pas prédominante, permettant ainsi l'existence de plus d'un état excité métastable. Dans le cas de terre rare, le couplage électron-phonon des transitions f-f est réduit car les électrons 4f ou 5f ne sont pas fortement impliqué dans la liaison métal-ligand. Ceci a pour conséquence une efficacité plus faible des processus de relaxation multiphononique. Le phénomène d'upconversion est ainsi plus commun et donc plus étudié dans les matériaux contenant des terres rares. Mais il existe des systèmes métaux de transitions et des combinaisons métaux de transitions/ terre rare présentant ce phénomène.

Il est toutefois nécessaire d'exciter en permanence le matériau pour que l'émission de lumière ait lieu.

Nanoparticules à luminescence persistante.

L'invention décrit un nouveau type de marqueurs utilisant des nanoparticules à luminescence persistante. Le mécanisme physique permettant une émission de photons plusieurs heures après la fin de l'excitation est complexe mais peut être décrit de façon simple par le schéma de la figure 13.

L'excitation qui se fait généralement par excitation lumineuse dans l'UV ou le visible mais peut se faire également par RX. Cet excitation provoque la formation d'un exciton (ie paire électron-trou) qui va se séparer (ce qui n'est pas possible dans le cas des quantums dots). Une partie de l'énergie captée va ainsi être « stockée » au niveau de pièges à électron. Cet électron piégé va ensuite être libéré par activation thermique pour se recombiner au niveau d'un émetteur avec émission d'un photon.

Ainsi, le matériau à luminescence persistante peut être vu comme un condensateur qui va se charger sous l'effet d'une excitation et qui va progressivement se décharger en émettant des photons. Ce mécanisme est donc particulièrement différent de tous ceux énoncés préalablement.

Les inventeurs ont ainsi développé des sondes luminescentes prometteuses, utilisables à la fois en imagerie *in vivo* pour le petit animal et *in vitro* pour le développement d'outils d'analyses pour les biologistes.

La présente invention a pour but d'utiliser des nanomatériaux à luminescence persistante (matériaux dont l'émission peut perdurer plusieurs heures après l'arrêt de l'excitation) pour l'imagerie optique *in vivo.* L'excitation du matériau peut ainsi être effectuée avant que celui-ci ne soit injecté dans l'organisme à étudier. Ceci permet d'éviter l'autofluorescence des tissus, point essentiel pour l'accroissement du rapport signal sur bruit.

Pour un matériau donné, la longueur d'onde d'émission dépend du dopant. Elle est donc relativement modulable et l'émission pourra être adaptée à la fenêtre de transparence des tissus, entre 600 et 1300 nm. Les nanomatériaux inorganiques à luminescence persistante offrent ainsi des avantages considérables pour leur utilisation *in vivo,* d'une part pour améliorer la qualité des images avec une imagerie des tissus profonds, et d'autre part, sous forme de particules nanométriques, le matériau est rendu injectable, le cas échéant par traitement de surfaces avec des entités biocompatibles tout en conservant ses qualités optiques. Plusieurs applications sont aujourd'hui envisagées : grâce à ces composés, il est ainsi possible de visualiser les zones de rupture de la barrière hématoencéphalique, les zones inflammatoires et tumorales, et aussi de visualiser la biodistribution des liposomes *in vivo* dans une stratégie de thérapie génique ou d'administration de médicaments utilisant des nanovecteurs.

Comme document de l'art antérieur, on peut citer l'article Jiang et al. (Journal of Alloys and Compounds 377 (2004) 211-215), qui a pour objet la propriété de luminescence persistante de $CaMgSi_2O_6$ dopé avec de l'europium et du dysprosium. Toutefois, ce document ne décrit pas ni ne suggère de procéder à l'excitation de ce composé puis de l'administrer *in vivo,* notamment à des fins d'imagerie optique. En effet, les études portant sur les matériaux à luminescence persistante concernent principalement la signalisation, l'éclairage, le marquage des textiles. Les documents US 6537829, US 2003/059635, US 2004/014060 et US 2003/180780, qui décrivent tous des nanoparticules inorganiques dopées luminescentes, mettent à contribution la fluorescence ou la propriété d'upconversion de ces composés dans des méthodes de diagnostic in vitro.

Aucun article relevé dans la littérature n'est concerné par l'objet de la présente invention.

Selon un premier aspect, la présente invention a pour objet une nanoparticule à luminescence persistante pour son utilisation in vitro en tant qu'agent de diagnostic, ladite nanoparticule émettant des photons à des longueurs d'onde comprises entre 400 et 1300 nm pendant au moins 0,01 seconde, après une excitation lumineuse à des longueurs d'onde comprises entre 100 et 800 nm, ou encore après excitation par des rayons X.

Les longueurs d'onde comprises entre 100 et 400 nm correspondent au domaine ultraviolet/ultraviolet extrême (VUV pour « vacuum ultraviolet »), celles entre 400 et 800 nm au domaine du visible, et celles entre 800 et 1300 correspondent au domaine infrarouge.

Les nanoparticules selon l'invention ne doivent pas émettre des photons à des longueurs d'onde inférieures à 400 nm, c'est-à-dire dans le domaine ultraviolet, car de telles longueurs d'onde sont absorbées par les molécules du vivant, ce qui entraîne leur détérioration progressive, la formation de composés phototoxiques et au final la destruction cellulaire.

D'autre part, les nanoparticules selon l'invention ne doivent pas émettre des photons à des longueurs d'onde supérieures à 1300 nm, c'est-à-dire dans le domaine infrarouge lointain, car les ondes électromagnétiques sont absorbées sous forme d'énergie thermique par l'ensemble des molécules (surtout par les molécules d'eau).

Les nanoparticules selon l'invention qui émettent des photons pendant moins de 0,01 seconde ne sont pas comprises dans la présente demande car il s'agit alors de fluorescence, et plus de luminescence persistante.

De préférence, la nanoparticule émet des photons pendant au moins 1 seconde, de manière avantageuse pendant au moins 1 minute, 30 minutes, 1 heure, voire au moins 10 heures.

Les temps de persistance sont évalués en suivant l'intensité de luminescence en fonction du temps après excitation. Des comparaisons claires de mesures des temps de persistance doivent être réalisées dans des conditions identiques en utilisant les mêmes systèmes de détection. L'expression « matériau à luminescence persistante » a été appliquée à des matériaux présentant une luminescence d'au moins 0,01 seconde jusqu'à plusieurs heures. Les matériaux à luminescence persistante, incluant des cristaux simples et des fibres de cristaux simples, peuvent présenter des temps de persistance de la luminescence supérieurs à environ 3 à 5 heures, supérieurs à environ 10 à 12 heures, ou supérieurs à environ 15 à 18 heures.

Le phénomène de luminescence persistante implique deux types de centres actifs : les émetteurs et les pièges (capteurs). Les émetteurs sont des centres capables d'émettre une radiation après excitation du centre. Les pièges n'émettent pas de radiation, mais stockent l'énergie de radiation et la libèrent progressivement à l'émetteur. Les centres émetteurs peuvent être créés par l'addition d'activateurs, c'est-à-dire des petites quantités d'atomes ou d'ions d'impureté ajoutés intentionnellement à la matrice hôte. Les co-activateurs sont des ions d'impuretés ou des défauts ponctuels (lacunes d'anions) ajoutés intentionnellement qui peuvent affecter (améliorer ou modifier) le temps de vie de l'émission du premier activateur. Par exemple, un co-activateur peut être ajouté pour former des centres de captage qui peuvent améliorer le temps de persistance du matériau à luminescence persistante.

Il est possible pour un ion de transférer l'énergie à un autre. Si deux ions différents sont impliqués dans l'énergie de transfert, l'ion transférant l'énergie est appelé un donneur tandis que l'ion recevant l'énergie est appelé l'accepteur ou activateur (G. Blasse et B.C. Grabmaier, 1994, « Luminescent materials », Springer-Verlag, Berlin, p. 91).

Les matériaux de la présente invention sont basés sur le dopage d'un ou de plusieurs émetteurs dans une matrice hôte. L'hôte (ou matrice) et l'(les) ion(s) émetteur(s) est(sont) choisi(s) pour fournir l'émission souhaitée ou la couleur de luminescence persistante et un rendement quantique élevé.

La présente invention est applicable à tout type d'espèce du règne animal, avantageusement aux vertébrés et plus particulièrement au petit animal (rongeurs), mais également à l'homme.

Dans la présente demande, on entend désigner par « nanoparticule » une particule dont la taille, définie telle que la plus grande dimension selon un axe, est de manière générale comprise entre 10 nm et 10 μm.

De préférence, la nanoparticule selon l'invention est comprise entre 25 nm et 1 μm, de manière encore préférée, entre 50 nm et 500 nm.

La nanoparticule à luminescence persistante peut ainsi être constituée à titre d'exemple non limitatif par un composé tel que $CdSiO_3 : Mn^{2+}$, $ZnGa_2O_4 : Mn^{2+}$, $ZnS:Cu$ ou $Y_2O_2S : Ti, Mg, Ca$. Elle peut être constituée par un composé du type silicate, aluminate, aluminosilicate, germanate, titanate, oxysulfure, phosphate ou vanadate, ledit composé comprenant au moins un oxyde de métal et étant dopé avec au moins un ion terre rare, et éventuellement avec au moins un ion d'un métal de transition (on peut citer comme exemple le manganèse ou le chrome trivalent). Elle peut encore être constituée par un sulfure comprenant au moins un ion métallique choisi parmi le zinc, le strontium et le calcium, dopé avec au moins un ion terre rare, et éventuellement avec au moins un ion d'un métal de transition. On peut également citer les oxydes de métaux, que l'on dope là encore avec au moins un ion terre rare, et éventuellement avec au moins un ion d'un métal de transition.

Cette liste de composés est non limitative, et l'homme du métier est en mesure de déterminer quels matériaux à luminescence persistante peuvent être utilisés dans le cadre de la présente invention

De préférence, la nanoparticule est constituée par un composé choisi dans le groupe constitué par :

- les silicates, les aluminates, les aluminosilicates, les germanates, les titanates, les oxysulfures, les phosphates et les vanadates, de tels composés comprenant au moins un oxyde de métal,
- les sulfures comprenant au moins un ion métallique choisi parmi le zinc, le strontium et le calcium, et
- les oxydes de métaux, ledit composé étant dopé avec au moins un ion terre rare, et éventuellement avec au moins un ion d'un métal de transition.

Comme exemple d'oxysulfures on peut citer les composés à base d'yttrium tels que les sulfures d'oxyde d'yttrium ($Y_2O_2S$,...). Les germanates peuvent être tels que $MGeO_3$ dans lequel M est le magnésium, le calcium ou le zinc, de préférence le magnésium ($Mg^{2+}$) et le calcium ($Ca^{2+}$), de tels germanates étant de préférence dopés avec les ions manganèse et un ion trivalent de la série des lanthanides. On peut encore citer les titanates tels que $MO\text{-}TiO_2$ dans lequel M est le magnésium ou le zinc, et les sulfures tels que le sulfure de zinc (ZnS), le sulfure de calcium (CaS) et le le sulfure de strontium (SrS).

Le métal de l'oxyde de métal peut être de tout type. De préférence, il est choisi parmi le magnésium, le calcium, le strontium, le baryum, le zinc, le cadmium, l'yttrium et le gallium.

Le métal de transition peut être de tout type. De préférence, le métal de transition est choisi parmi le manganèse, le chrome et le titane ($Mn^{2+}$, $Cr^{3+}$, $Ti^{4+}$,...).

L'ion terre rare peut être de tout type. De préférence, l'ion terre rare est choisi parmi les ions europium, ytterbium, cérium, samarium, praséodyme, dysprosium, néodyme, holmium, terbium, thulium et erbium. L'ion terre rare se trouve sous sa forme trivalente ($Ce^{3+}$, $Dy^{3+}$, $Nd^{3+}$, $Ho^{3+}$, $Er^{3+}$,...) sauf pour l'europium, le samarium et l'ytterbium, qui peuvent se trouver également sous leur forme divalente ($Eu^{2+}$, $Sm^{2+}$ et $Yb^{2+}$).

De préférence, la nanoparticule est constituée par un silicate comprenant un oxyde de métal dopé avec au moins un ion terre rare et au moins un ion d'un métal de transition. De manière encore préférée, le silicate est dopé avec les ions manganèse, europium et dysprosium.

De manière encore plus préférée, la nanoparticule est constituée par un composé choisi dans le groupe constitué par les silicates $ZnMgSi_2O_6$, $CaMgSi_2O_6$ et $MgSiO_3$, de tels silicates étant dopés avec les ions manganèse, europium et dysprosium, et $Sr_2MgSi_2O_7$ dopé avec les ions europium et dysprosium.

Selon un autre mode de réalisation particulièrement préféré, la nanoparticule est constituée par le silicate $Ca_{0.2}Zn_{0.9}Mg_{0.9}Si_2O_6$ dopé $Eu^{2+}$, $Dy^{3+}$, $Mn^2$.

De manière générale, les nanoparticules selon l'invention peuvent être administrées sans être fonctionnalisées dans l'organisme ou le tissu. En particulier, injectées par voie intra-artérielle ou intraveineuse, elles permettent une imagerie du réseau vasculaire, en particulier au niveau des poumons et du foie. Elles permettent également une imagerie fonctionnelle du foie. Un des objectifs est l'imagerie des zones tumorales ou inflammatoires qui sont hypervascularisées. Dans le cas des zones tumorales, une détection précoce d'un cancer tel que le cancer du sein est possible.

Selon un mode de réalisation particulier, la nanoparticule selon l'invention est fonctionnalisée par enrobage et/ou greffage de ligand permettant une liaison avec une substance d'intérêt biologique ou chimique.

Par substance d'intérêt biologique ou chimique on entend désigner toute substance dont on cherche à déterminer la biodistribution dans un organisme ou dans un tissu. La substance d'intérêt peut être par exemple une molécule chimique telle qu'un principe actif ou bien une substance toxique. Il peut encore s'agir d'un récepteur extracellulaire, d'une hormone, d'un anticorps ou d'un antigène, d'une protéine, d'une toxine telle qu'une toxine bactérienne éventuellement sous sa forme atténuée ou d'un acide nucléique, d'un virus ou autre agent pathogène (bactérie,...).

Les termes protéine, polypeptide ou peptide désignent indifféremment une séquence d'acides aminés ou, pour leurs dérivés, un composé contenant une séquence d'acides aminés. De même, les termes acide nucléique, séquence nucléique ou d'acide nucléique, polynucléotide, oligonucléotide, séquence de polynucléotide, séquence nucléotidique, sont employés indifféremment pour désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADN. Ces acides nucléiques sont isolés de leur environnement naturel, et sont naturels ou artificiels.

Pour déterminer la biodistribution d'une substance d'intérêt biologique ou chimique dans un organisme ou dans un tissu, il est nécessaire de fonctionnaliser la nanoparticule par enrobage et/ou greffage d'un ligand capable de se lier à la substance, le cas échéant présente au niveau tissulaire de l'organe d'intérêt.

Les méthodes d'enrobage sont bien connues de l'homme de l'art. Par exemple, l'enrobage peut être effectué par liaison avec des molécules portant des groupes phosphates, carboxylates ou thiols, ou encore par hétéroprécipitation de silice, d'aminosilane, ou de préférence de triéthoxyaminopropylsilane. L'enrobage effectué avec du triéthoxyaminopropylsilane offre l'avantage qu'une seule couche se forme, sans polymérisation vers l'extérieur entraînant l'augmentation de la taille des nanoparticules.

De même, les méthodes de greffage (ou couplage) du ligand sont bien connues de l'homme de l'art. Il s'agit en général d'un couplage par liaison covalente, par affinité, par adsorption passive ou forcée. Dans le cas d'un couplage par liaison covalente, les nanoparticules sont porteuses de groupements chimiques capables de réagir avec un autre groupement chimique porté par le ligand pour former une liaison covalente.

Comme exemple de groupements chimiques pouvant être présents à la surface des nanoparticules on peut citer, mais sans s'y limiter, les groupements carboxyle, amino, aldéhyde et époxy.

On peut également utiliser l'interaction par affinité, qui est généralement mise en oeuvre par deux partenaires d'un couple de liaison à haute affinité tels que notamment, mais sans s'y limiter, les couples (poly) carbohydrate/lectine, biotine ou composés biotinilés/avidine ou streptavidine, récepteur /ligand spécifique, antigène ou haptène/anticorps, etc...

Le greffage des nanoparticules enrobées peut également être réalisé soit directement, soit en utilisant des bras espaceurs encore désignés sous les termes "linker" ou "spacer".

Le couplage par adsorption passive ou forcée est connu de l'homme de l'art. On pourra utiliser par exemple la BSA-biotine (Bovin Serum Albumin) (Sigma, Lyon, FR - Réf. A-8549).

De préférence, l'enrobage est réalisé par précipitation à la surface de triéthoxyaminopropylsilane.

Selon un autre mode préféré, on greffe du polyéthylène glycol (PEG) à des fins de furtivité (pour obtenir un temps de circulation dans l'organisme plus important). On procède généralement de la manière suivante : le PEG est d'abord couplé au ligand, puis le produit du couplage est greffé sur la nanoparticule.

De manière encore plus préférée, la nanoparticule selon l'invention est fonctionnalisée par précipitation à la surface de triéthoxyaminopropylsilane puis greffage de méthoxy-PEG$_{5000}$-COOH permettant une liaison avec la substance d'intérêt biologique ou chimique.

En outre, le toluène peut être remplacé par du diméthylformamide, permettant ainsi une meilleure dispersion des particules. Les particules peuvent être fonctionnalisées par des groupements carboxylates (par réaction d'anhydride diglycolique sur les particules aminées) mais aussi des groupements thiol par réaction directe avec du 3-mercaptopropyl-triéthoxysilane. Le greffage de polyéthylène glycol (PEG) peut être directement réalisé par couplage peptidique (cf. Exemple 5). Les Inventeurs ont également réussi à greffer différentes molécules chimiques à la surface des nanoparticules (biotine, peptide).

Selon un mode de réalisation préféré, la nanoparticule selon l'invention est fonctionnalisée par des groupements carboxylates, thiol ou amine libre.

Selon un mode de réalisation particulier, la présente invention a pour objet la nanoparticule telle que définie précédemment pour son utilisation en tant qu'agent de diagnostic destiné à l'imagerie optique *in vivo.*

De manière préférée, ladite nanoparticule est excitée avant l'administration. En effet, l'absence d'excitation optique du sujet permet d'éliminer l'autofluorescence des tissus, point essentiel pour l'accroissement du rapport signal/bruit.

De préférence, l'agent de diagnostic est administré dans un organisme à étudier par injection par voie intraveineuse, intraartérielle ou intramusculaire.

De préférence, l'agent de diagnostic est destiné à l'imagerie de la vascularisation de l'organisme ou des organes réticulo-endothéliaux (foie, rate).

De manière générale, on préfèrera les nanoparticules qui émettent des photons à des longueurs d'onde comprises entre 600 et 1300 nm (cf. Fig 1) pour l'imagerie des tissus profonds (organes, etc...).

L'imagerie vasculaire est, par exemple, intéressante pour la mise en évidence de processus angiogéniques dans l'inflammation chronique, la croissance tumorale ou la localisation des métastases. Les études de biodistribution sont, par ailleurs, indispensables pour déterminer les rapports d'accumulation d'un agent vectorisé ou non par des formes particulaires au niveau des tissus cibles.

De manière encore préférée, l'agent de diagnostic est destiné à l'imagerie de zones tumorales, inflammatoires, de la rétine, lesdites zones étant susceptibles d'être hypervascularisées, ou encore des zones de rupture de la barrière hématoencéphalique.

Selon un autre mode de réalisation, l'agent de diagnostic est destiné à l'imagerie de zones hypovascularisées telles que dans le cas d'une ischémie cérébrale ou cardiaque, ou dans le cas d'un trauma crânien.

Selon encore un autre mode de réalisation, l'agent de diagnostic permet de mimer la biodistribution de liposomes ou de nanovecteurs *in vivo* dans une stratégie de thérapie génique.

Selon un deuxième aspect, la présente description a pour objet un procédé pour la détection ou la quantification *in vitro* d'une substance d'intérêt biologique ou chimique dans un échantillon, qui comprend les étapes suivantes :

1) la mise en contact dudit échantillon avec une solution contenant des nanoparticules à luminescence persistante préalablement fonctionnalisées de manière à former un complexe entre la substance et les nanoparticules, et

2) la détection ou la quantification dudit complexe formé,

caractérisé en ce que lesdites nanoparticules sont constituées par un composé choisi dans le groupe constitué par :

- les silicates, les aluminosilicates, les germanates, les titanates, les oxysulfures, les phosphates et les vanadates, de tels composés comprenant au moins un oxyde de métal,
- les sulfures comprenant au moins un ion métallique choisi parmi le zinc, le strontium et le calcium, et
- les oxydes de métaux,

ledit composé étant dopé avec au moins un ion terre rare, et éventuellement avec au moins un métal de transition.

On entend désigner par échantillon dans le procédé selon la présente invention tout échantillon susceptible de contenir la substance d'intérêt biologique ou chimique que l'on souhaite détecter ou quantifier.

Comme exemple d'oxysulfures on peut citer les composés à base d'yttrium tels que les sulfures d'oxyde d'yttrium ($Y_2O_2S$,...). Les germanates peuvent être tels que $MO-GeO_2$ dans lequel M est le magnésium, le calcium ou le zinc, les titanates tels que $MO-TiO_2$ dans lequel M est le magnésium ou le zinc, et les sulfures tels que le sulfure de zinc (ZnS), le sulfure de calcium (CaS) et le sulfure de strontium (SrS).

De préférence, le métal de l'oxyde de métal est choisi parmi le magnésium, le calcium, le strontium, le baryum, le zinc, le cadmium, l'yttrium et le gallium.

De préférence, le métal de transition est choisi parmi le manganèse, le chrome et le titane ($Mn^{2+}$, $Cr^{3+}$, $Ti^{4+}$,...).

De préférence, l'ion terre rare est choisi parmi les ions europium, ytterbium, cérium, samarium, praséodyme, dysprosium, néodyme, holmium, terbium, thulium et erbium. L'ion terre rare se trouve sous sa forme trivalente ($Ce^{3+}$, $Dy^{3+}$, $Nd^{3+}$, $Ho^{3+}$, $Er^{3+}$,...) sauf pour l'europium, le samarium et l'ytterbium, qui peuvent se trouver également sous leur forme divalente ($Eu^{2+}$, $Sm^{2+}$ et $Yb^{2+}$).

De manière encore plus préférée, les nanoparticules sont constituées par un silicate comprenant un oxyde de métal dopé avec au moins un ion terre rare et au moins un métal de transition.

De manière préférée entre toutes, les nanoparticules sont constituées par un composé choisi dans le groupe constitué par les silicates $ZnMgSi_2O_6$, $CaMgSi_2O_6$ et $MgSiO_3$, de tels silicates étant dopés avec les ions manganèse, europium et dysprosium, et $Sr_2MgSi_2O_7$ dopé avec les ions europium et dysprosium.

Selon un mode de réalisation particulièrement préféré, le procédé pour la détection ou la quantification *in vitro* est caractérisé en ce que les nanoparticules sont constituées le silicate $Ca_{0.2}Zn_{0.9}Mg_{0.9}Si_2O_6$ dopé $Eu^{2+}$, $Dy^{3+}$, $Mn^{2+}$.

Les nanoparticules peuvent être fonctionnalisées comme décrit ci-dessus.

Selon un dernier aspect, la description a pour objet un kit de diagnostic comprenant une solution contenant des nanoparticules à luminescence persistante constituées par un composé choisi dans le groupe constitué par :

- les silicates, les aluminates, les germanates, les titanates, les oxysulfures, les phosphates et les vanadates, de tels composés comprenant au moins un oxyde de métal,
- les sulfures comprenant au moins un ion métallique choisi parmi le zinc, le strontium et le calcium, et
- les oxydes de métaux,

ledit composé étant dopé avec au moins un ion terre rare, et éventuellement avec au moins un métal de transition.

De préférence, le kit est caractérisé en ce que les nanoparticules sont constituées par un silicate comprenant un oxyde de métal dopé avec au moins un ion terre rare et au moins un métal de transition. De manière encore préférée, le kit est caractérisé en ce que les nanoparticules sont constituées par un composé choisi dans le groupe constitué par les silicates $ZnMgSi_2O_6$, $CaMgSi_2O_6$ et $MgSiO_3$, de tels silicates étant dopés avec les ions manganèse, europium et dysprosium, et $Sr_2MgSi_2O_7$ dopé avec les ions europium et dysprosium. Selon un autre mode de réalisation préféré, le kit est caractérisé en ce que les nanoparticules sont constituées le silicate $Ca_{0.2}Zn_{0.9}Mg_{0.9}Si_2O_6$ dopé $Eu^{2+}$, $Dy^{3+}$, $Mn^2$.

Les exemples et figures qui suivent sont destinés à illustrer l'invention sans aucunement en limiter la portée.

**LEGENDE DES FIGURES**

**Figure 1 : 1A** Coefficient d'extinction molaire de l'hémoglobine en fonction des longueurs d'onde (d'après W. B. Gratzer et N. Kollias) ; **1B** Coefficient d'extinction de l'eau en fonction des longueurs d'onde (d'après G. M. Hale, M. R. Querry. Applied Optics,12 (1973) 555-563)

**Figure 2 :** Décroissance typique du signal de luminescence des composés synthétisés

**Figure 3 :** Spectre d'excitation et d'émission de $ZnMgSi_2O_6$ : $Eu^{2+}$ $Dy^{3+}$ $Mn^{2+}$

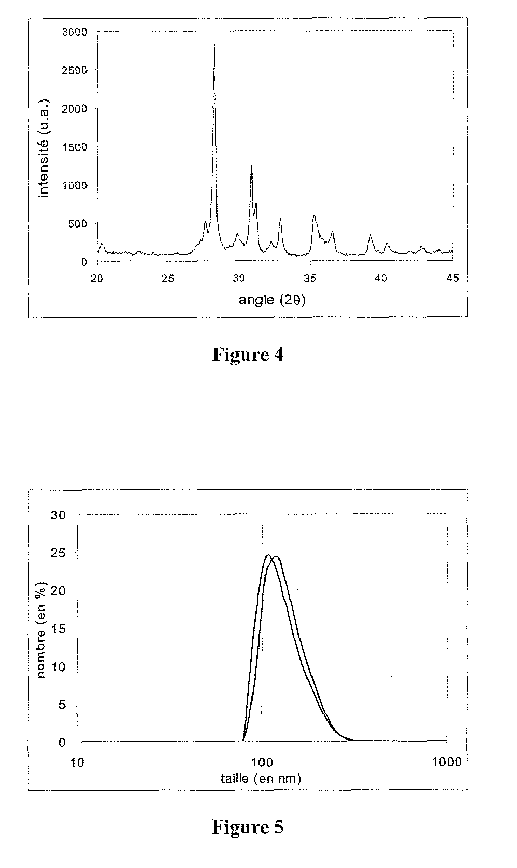

**Figure 4 :** Diagramme de diffraction des RX de $ZnMgSi_2O_6$ : $Eu^{2+}$ $Dy^{3+}$ $Mn^{2+}$

**Figure 5 :** Distribution de taille obtenue par diffusion quasi-élastique de la lumière des particules de $ZnMgSi_2O_6$ : $Eu^{2+}$ $Dy^{3+}$ $Mn^{2+}$

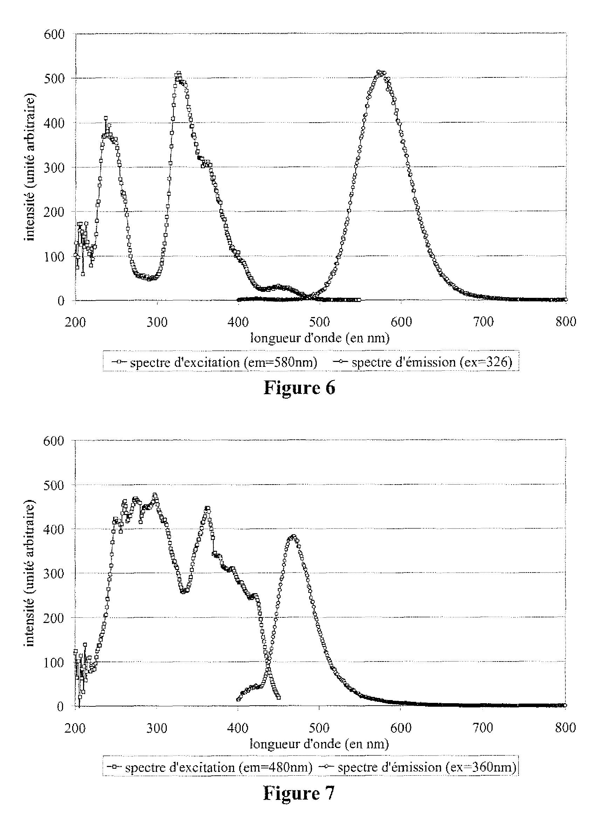

**Figure 6 :** Spectre d'excitation et d'émission de $CaMgSi_2O_6$ : $Eu^{2+}$ $Dy^{3+}$ $Mn^{2+}$

**Figure 7 :** Spectre d'excitation et d'émission de

$Sr_2MgSi_2O_7 : Eu^{2+} Dy^{3+}$

**Figure 8 :** Dynamique des nanoparticules de $ZnMgSi_2O_6$ dopé Eu, Mn, Dy après injection intraveineuse

**Figure 9 :** Dynamique des nanoparticules de $ZnMgSi_2O_6$ dopé Eu, Mn, Dy recouvertes de methoxy-$PEG_{5000}$ après injection intraveineuse **Figure 10 :** Diagramme de Jablonski

**Figure 11** : Spectres de luminescence et durées de vie des chélates de lanthanides proposés dans le système DELFIA® par Perkin Elmer.

**Figure 12 :** Schéma expliquant le phénomène d'upconversion dans les matériaux

**Figure 13 :** Schéma simplifié de la luminescence persistante dans les matériaux

**Figure 14 :** Propriétés des nanoparticules $Ca_{0.2}Zn_{0.9}Mg_{0.9}Si_2O_6$ dopés $Eu^{2+}$ (0,5%),$Dy^{3+}$(1,0%), $Mn^{2+}$ (2,5%)

(14A) Diagramme de diffraction aux rayons X (Intensité a.u. en ordonnées).

(14B) Images obtenues en microscopie électronique en transmission (échelle : 200 nm)

(14C) Spectre d'excitation (Intensité normalisée en ordonnées)

(14D) Spectre d'émission de luminescence persistante (Intensité normalisée en ordonnées)

(14E) Décroissance de luminescence du composé (Intensité a.u. en ordonnées).

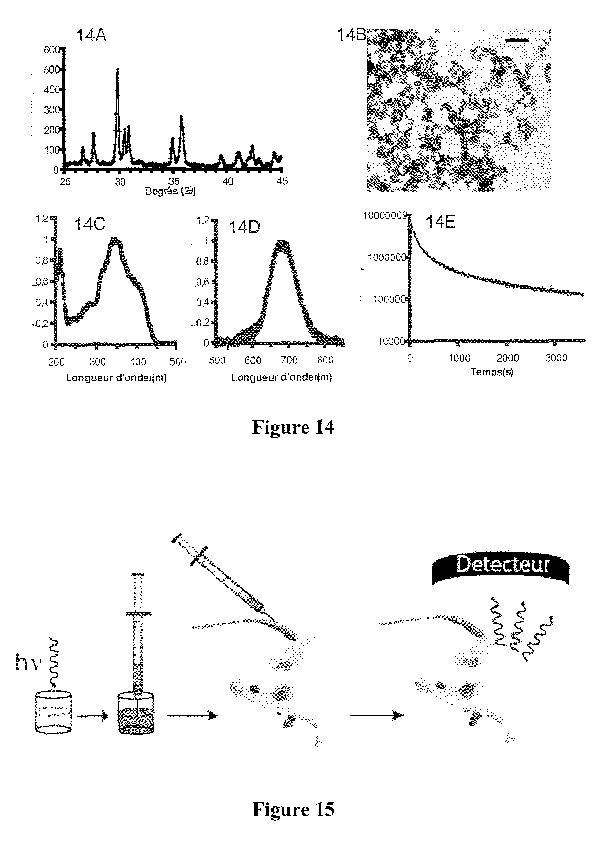

**Figure 15 :** Principes des expériences *in vivo* utilisant les propriétés de luminescence persistante des nanoparticules synthétisées

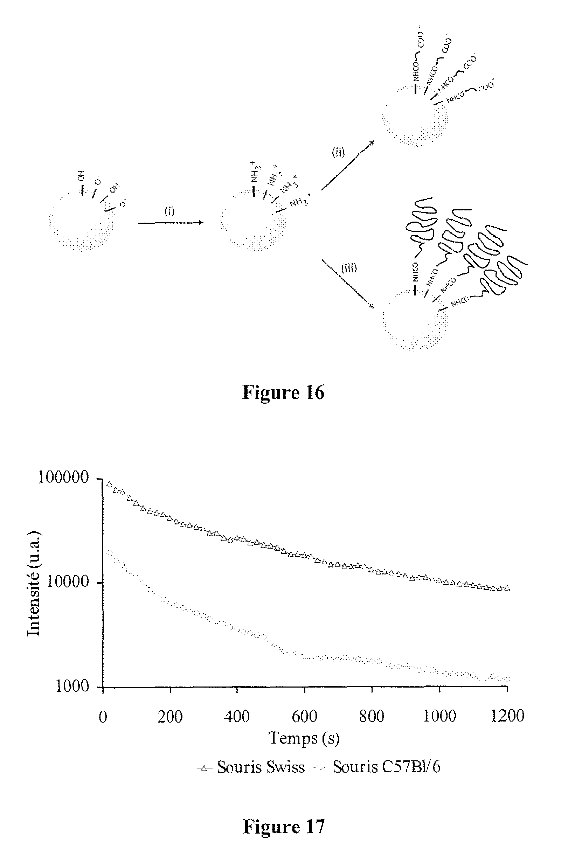

**Figure 16 :** Schéma de fonctionnalisation possible pour obtenir des particules portant des charges de surfaces différentes (i) APTES (ii) anhydride diglycolique (iii) mPEG-COOH, BOP,$Et_3N$

**Figure 17 :** Intensité du signal obtenue lors d'une injection systémique de nanoparticules à luminescence persistante chez deux types de souris (souris Swiss et souris C57B1/6)

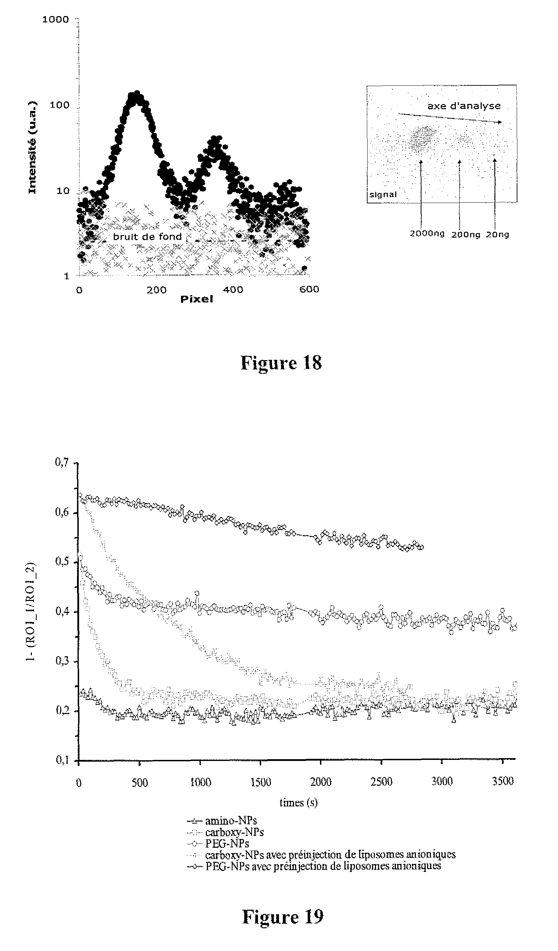

**Figure 18 :** Analyse du signal obtenu lors de trois injections sous-cutanées de nanoparticules (2 $\mu$g, 200 ng et 20 ng) sur le dos d'une souris.

Image du signal obtenu montrant l'axe d'analyse choisi, la localisation et la quantité de nanoparticules injectées.

**Figure 19 :** Analyse de la répartition d'intensité lumineuse selon le type de nanoparticules injectées (amino-NPs, carboxy-NPs et PEG-NPs) avec ou sans préinjection d'une suspension de liposomes anioniques. ROI_1 correspond au signal provenant de la région d'intérêt couvrant les poumons, le foie et la rate. ROI_2 correspond au signal provenant de la région d'intérêt couvrant l'ensemble de la souris.

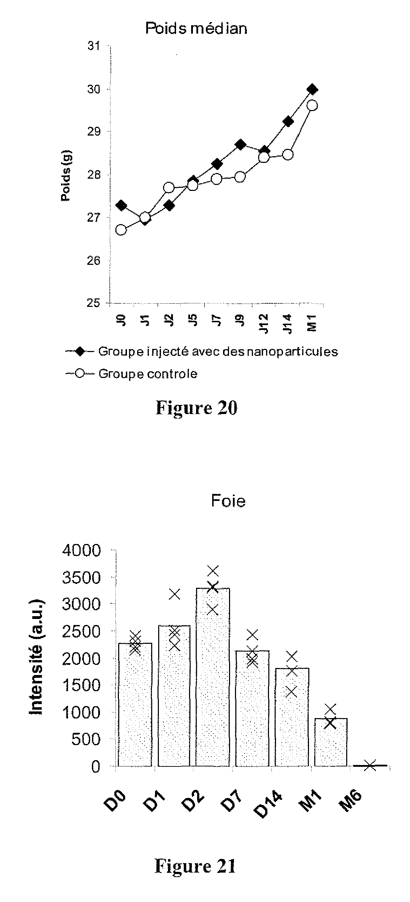

**Figure 20 :** Poids médian du groupe de souris injecté par 1 mg de nanoparticules et du groupe contrôle (injecté avec du sérum physiologique).

**Figure 21 :** Quantité de nanoparticules retrouvées au cours du temps dans le foie (analyse faite sur un groupe de 4 souris pour chaque temps).

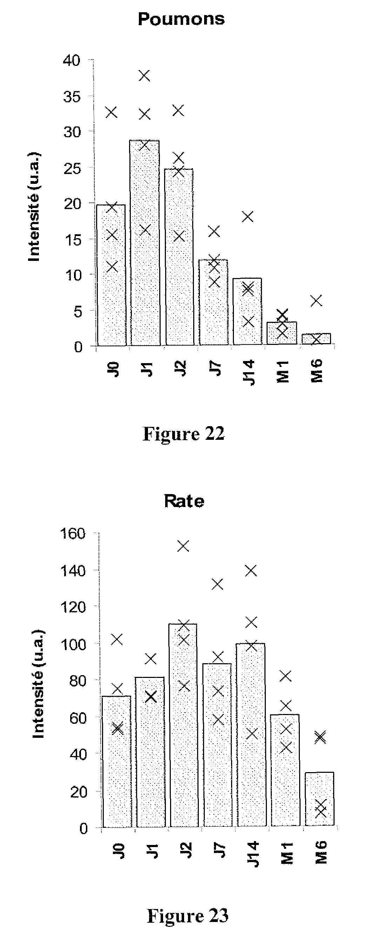

**Figure 22 :** Quantité de nanoparticules retrouvées au cours du temps dans les poumons (analyse faite sur un groupe de 4 souris pour chaque temps).

**Figure 23 :** Quantité de nanoparticules retrouvées au cours du temps dans la rate (analyse faite sur un groupe de 4 souris pour chaque temps).

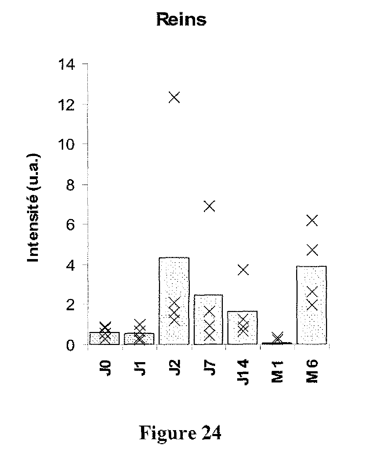

**Figure 24 :** Quantité de nanoparticules retrouvées au cours du temps dans les reins (analyse faite sur un groupe de 4 souris pour chaque temps).

**EXEMPLES**

**EXEMPLE 1 : SYNTHESE GEL DU COMPOSE $ZNMGSI_2O_6$ DOPE EU, MN, DY**

1) Synthèse du gel

Dans un ballon, on dissout dans 4mL d'eau acidifiée à pH 2 (par addition d'acide nitrique) 610mg ($4,98.10^{-3}$ mol) de Chlorure de Zinc, 1,150mg ($4,98.10^{-3}$ mol) de Nitrate de Magnésium hexahydrate, 20mg ($4,98.10^{-5}$ mol) de Nitrate d'Europium hexahydrate, 58.6mg ($1,49.10^{-4}$ mol) de Nitrate de Dysprosium hexahydrate et 29.4 mg ($1.34.10^{-4}$mol) de Chlorure de Manganèse tetrahydrate.

Sous agitation, on ajoute 2mL ($8,96.10^{-3}$mol) de tetraethoxysilane. On agite fortement à l'aide d'un vortex pour homogénéiser la solution puis on laisse sous agitation à température ambiante pendant 3h.

2) Séchage

Lorsque la solution est totalement monophasée (après environ trois heures d'agitation), on place le ballon à l'étuve à 60°C pendant quatre heures. On obtient alors un gel translucide. On sèche ensuite les échantillons à l'étuve à 110°C pendant 12h.

3) Chauffage

On chauffe ensuite les matériaux dans un four sous atmosphère réductrice (on utilise du Noxal : 90% Ar, 10% $H_2$). Le chauffage s'effectue en trois étapes, la montée en température : 20°C/min jusqu'à 1150°C, un palier de 10h (1150°C constant) puis une descente en température de 10°C/min jusqu'à température ambiante.

Après ce premier chauffage, un chauffage sous air suivi d'un chauffage sous atmosphère réductrice peut être nécessaire pour améliorer la luminescence.

La synthèse gel des composés $CaMgSi_2O_6$ dopé Eu, Mn, Dy, $MgSiO_3$ dopé Eu, Mn, Dy, et $Sr_2MgSi_2O_7$ dopé Eu, Dy est effectuée de manière analogue.

**EXEMPLE 2 : PROPRIETES PHYSIQUES DES COMPOSES EXEMPLIFIES**

Les composés $ZnMgSi_2O_6$, $CaMgSi_2O_6$,

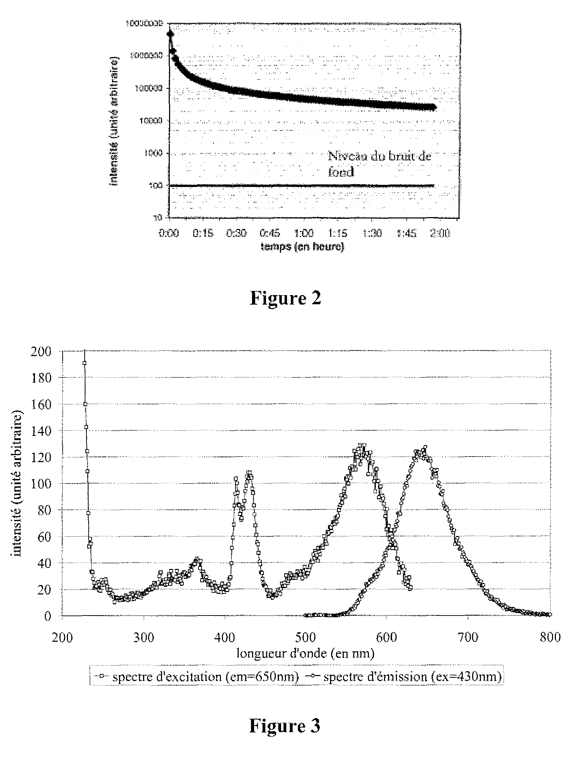

MgSiO$_3$ dopés Eu, Mn, Dy, et Sr$_2$MgSi$_2$O$_7$ dopé Eu, Dy obtenus par synthèse sol-gel comme détaillé ci-dessus (exemple 1), possèdent une luminescence persistante de plusieurs heures (supérieur à deux heures, voir Figure 2). Toutefois, ces compositions relatives peuvent être modifiées pour améliorer les propriétés de luminescence de composés. Grâce à la synthèse sol-gel décrite ci-dessus, les composés gardent une taille mésoscopique (taille entre 10 nm et 1μm, préférentiellement entre 50 et 500 nm). Il est ainsi possible de les redisperser dans une solution aqueuse pour obtenir une solution luminescente injectable.

Certains composés exemplifiés possèdent une luminescence persistante à des longueurs d'onde particulièrement intéressantes pour les milieux biologiques (voir Figure 3). Le mécanisme de luminescence persistante avec un transfert de l'excitation sur le manganèse émet autour de 650 nm dans ces matrices. Grâce à cette émission longue dans le rouge, il est ainsi possible de réaliser une imagerie optique *in vivo,* la zone de transparence des tissus biologiques étant comprise entre 600 et 1300 nm.

**EXEMPLE 3: PRECIPITATION DE TRIETHOXYAMINOPROPYLSILANE PUIS GREFFAGE DE METHOXY-PEG$_{5000}$-COOH A LA SURFACE DE NANOPARTICULES A LUMINESCENCE PERSISTANTE.**

Prétraitement de surface :

Dans un ballon contenant une solution aqueuse de hydroxyde de sodium (5mM), on disperse 75 mg de nanoparticules. On agite la suspension à température ambiante pendant 3h. Puis, après addition d'acide chlorhydrique (1M) afin de revenir à pH neutre, on centrifuge la suspension (4500rpm,10min) pour enlever le surnageant. On sèche alors la poudre récupérée à l'étuve.

Précipitation de triéthoxyaminopropylsilane :

Dans un ballon contenant 5mL de toluène anhydre et 90μL de triéthoxyaminopropylsilane, on disperse 75mg de nanoparticules préalablement traitées. On laisse sous agitation pendant 4h à 80°C. On centrifuge ensuite la suspension (4500rpm,10min) pour enlever le surnageant. Pour laver la solution, on redisperse alors la poudre dans 5mL de toluène anhydre puis on centrifuge. Cette opération de lavage est ainsi répétée trois fois. La poudre est finalement séchée à l'étuve.

Un test caractéristique des amines primaires à l'acide 2,4,6 trinitrobenzène sulfonique se révèle positif sur les nanoparticules.

Greffage methoxy-PEG-COOH activé sur les nanoparticules :

Dans un ballon contenant 5mL de dichlorométhane, on dissout 250mg (5.10$^{-4}$ mol) de méthoxy-

PEG$_{5000}$ COOH . On ajoute 5.8mg de N-hydroxysuccinimide (5.10$^{-4}$ mol) et 10.3mg de dicyclohexylcarbodiimide (5.10$^{-5}$ mol). L'ensemble est laissé sous agitation à température ambiante pendant 2h

On évapore ensuite le solvant et on précipite le polymère activé dans de l'éther diéthylique. Le précipité ainsi formé est lavé à l'éther puis redissout ensuite dans 5mL de dichlorométhane.

On ajoute dans cette solution 15μL de triéthylamine et 50mg de nanoparticules recouvertes d'une couche d'aminosilane. On laisse le milieu réactionnel sous agitation pendant 3 heures.

On évapore ensuite le solvant et on redisperse les nanoparticules dans de l'eau. Après centrifugation (4500rpm,10min) et lavage à l'eau, on récupère les nanoparticules PEGylés que l'on sèche à l'étuve.

**EXEMPLE 4 : EXPERIENCES REALISEES**

Au vu des premiers résultats physiques de la poudre synthétisée, les inventeurs ont réalisé les premières expériences *in vitro* (prélèvement d'organes et observation sur coupes) et *in vivo* avec ces composés à luminescence persistante. Les injections *in vivo* ont été réalisées sur une souris SWISS avec une solution de matériau luminescent à une concentration de 30 mg par mL. Les expériences *in vivo* ont été réalisées en respectant les principes de bonnes pratiques de laboratoires.

L'excitation de la solution se fait avant injection par irradiation sous une lampe UV classique sans filtre. Les poudres n'ont pas subi de fonctionnalisation particulière (simple broyage en milieu acide (HCl) pour obtenir des charges de surface rendant les nanoparticules redispersables en milieu aqueux). Il est à noter que les composés restent luminescents dans les milieux biologiques (ce qui n'est pas le cas pour tous les matériaux à luminescence persistante. Pour ces autres composés, il est nécessaire d'enrober les nanoparticules). Les images ont été réalisées à l'aide de la caméra PhotoImager de Biospace Mesures.

Après injection intramusculaire, les résultats montrent que les matériaux exemplifiés, préalablement irradiés sous une lampe UV, sont toujours luminescents après injection dans l'animal. La luminescence est suffisamment importante pour traverser plusieurs millimètres de tissus classiquement étudiés en biologie. La localisation forte du signal est conforme aux attentes, les particules injectées en intramusculaire n'étant normalement pas emportées par la circulation sanguine.

Les injections en intraveineuse ont été réalisées au niveau de la queue de la souris. Les particules sont ainsi immédiatement emportées par la circulation sanguine. Les particules étant de taille nanométrique, elle ne se bloquent pas dans les poumons et donc se retrouvent naturellement dans le foie.

Le signal est suffisant pour obtenir une imagerie optique *in vivo* des particules plus de trente minutes après l'injection.

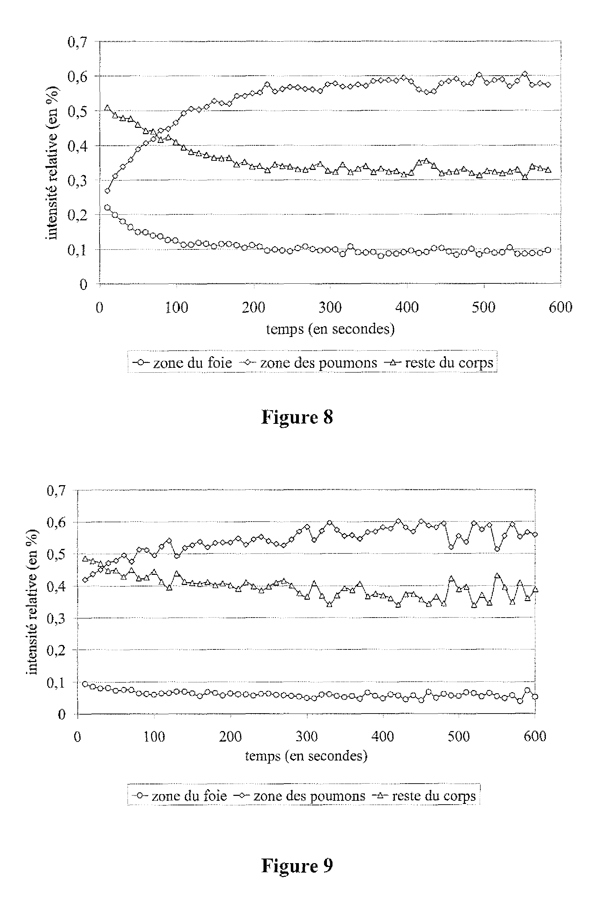

La figure 8 permet de rendre compte de la dynamique des particules in vivo des particules non recouvertes de PEG après injection intraveineuse. La figure 9, quant à elle, montre le comportement différent quand un greffage de PEG a été réalisé à la surface des nanoparticules. On y observe en effet une très faible rétention dans les poumons et augmentation de la luminescence provenant des zones autres que le foie et les poumons.

## EXEMPLE 5 : NOUVEAU COMPOSE $CA_{0.2}ZN_{0.9}MG_{0.9}SI_2O_6$ DOPE $EU^{2+}$, $DY^{3+}$, $MN^{2+}$

Afin d'optimiser l'émission dans l'infrarouge et la préparation de nanoparticules, les Inventeurs ont sélectionné le matériau $Ca_{0.2}Zn_{0.9}Mg_{0.9}Si_2O_6$ dopé $Eu^{2+}$, $Dy^{3+}$, $Mn^{2+}$. Les caractéristiques de ce matériau sont montrées à la Figure 14.

Les matériaux à luminescence persistante sont généralement synthétisés par voie solide, cette technique produisant des particules de taille micrométrique. Une approche par voie Sol-Gel a été développée pour réduire la taille des particules. Brièvement, à une solution d'eau acidifiée à pH~2 est ajoutée les différents sels dans les proportions désirées (chlorure de zinc, chlorure de calcium, nitrate de magnésium, chlorure d'europium, chlorure de dysprosium et chlorure de manganèse pour le composé $Ca_{0.2}Zn_{0.9}Mg_{0.9}Si_2O_6$ dopé $Eu^{2+}$, $Dy^{3+}$, $Mn^{2+}$). Après dissolution des sels, le tetraéthoxysilane (TEOS) est ajouté rapidement. La solution est mise sous agitation jusqu'à ce que la solution devienne limpide. Ceci est dû à l'hydrolyse du TEOS. La solution transparente est alors chauffée à 70°C jusqu'à gélification. Le gel est ensuite séché à 110°C pendant environ 20h puis calciné à 1050°C pendant 10h sous atmosphère réductrice afin de réduire $Eu^{3+}$ en $Eu^{2+}$. Cette réduction est essentielle pour obtenir dans ce matériau la propriété de luminescence persistante. Le matériau cristallin (Fig. 14A) obtenu est alors broyé à l'aide d'un mortier et d'un pilon. Les plus petites particules sont alors sélectionnées par sédimentation sélective. Une analyse par microscopie électronique a montré que les nanoparticules (NPs) présentaient une distribution en taille relativement homogène avec un diamètre de particules compris entre 50 et 100 nm (Fig. 14B).

Dans ce matériau, les pièges sont créés par l'introduction d'une faible quantité de $Dy^{3+}$, alors que $Mn^{2+}$ est le centre émetteur, recevant l'énergie issue des recombinaisons électron-trou. Les ions terres rares servent d'accepteur primaire d'énergie, laquelle est libérée de façon thermique et transmise au manganèse pendant plusieurs heures. La symétrie et le champ cristallin autour du $Mn^{2+}$ est responsable de l'émission dans le rouge et le proche infrarouge du manganèse dans le composé synthétisé. Cette émission correspond à la transition de l'état excité $^4T_1(^4G)$ vers l'état fondamental $^6A_1(^6S)$. Comme montré en Figure 14D, le spectre d'émission de luminescence persistante est assez large, avec un maximum d'intensité autour de 690 nm. Cette bande d'émission est particulièrement intéressante puisqu'elle se trouve dans la zone de transparence des tissus. La bande large d'excitation dans l'UV (Fig. 14C) ne pouvant pas être attribuée seulement aux bandes d'absorption du manganèse, la luminescence persistante observée est bien due à un transfert d'énergie à l'intérieur du matériau. Cette caractéristique de larges bandes d'excitation dans l'UV permet l'utilisation de lampes UV de laboratoires pour l'excitation des nanoparticules.

Pour l'acquisition du signal après suppression de la source d'excitation, un détecteur de type CCD (« charge coupled device ») peut être utilisé (PhotonImager, Biospace) sans système d'illumination externe. Lorsque les nanoparticules sont gardées dans le noir, la décroissance d'intensité lumineuse est typique d'un matériau à luminescence persistante et perdure pendant plus de 24 heures. La cinétique de décroissance (Fig. 14E) peut être approximée par une loi puissance $I \sim I_0 \times t^{-n}$ ($n=0.96$, $R^2=0.996$) pour les temps supérieurs à 100 s. A la connaissance des Inventeurs, c'est la première fois que des particules ayant ces propriétés spectrales et ces tailles nanométriques sont synthétisées.

Les Inventeurs n'ont pu trouver dans la littérature aucune utilisation de composés à luminescence persistante pour l'imagerie in vivo. Ils ont tout d'abord démontré que le niveau de luminescence produit par les nanoparticules après la fin de l'excitation était suffisant pour donner lieu à un signal localisable sous quelques mm de tissus, comme dans des expériences d'injections sous-cutanée ou intramusculaire. Une suspension de nanoparticules a ainsi été injectée en sous-cutané dans le dos d'une souris Swiss non préalablement rasée. Pour l'excitation avant injection, les nanoparticules en suspension ont été exposées directement à une lampe UV de 6 Watt pendant 5 min à une distance de 2 cm (Fig. 15). Afin de tester la plus faible dose détectable, 20 µL de suspensions à différentes concentrations de nanoparticules (100, 10, 1 µg/mL) ont été injectées à trois endroits différents du dos de la souris. Les deux doses les plus importantes (correspondant respectivement à 2 µg et 200 ng de nanoparticules) ont été facilement détectées. La dose administrée la plus faible (20 ng) a également donné un signal détectable avec un rapport signal sur bruit satisfaisant car supérieur à 5.

Afin de confirmer la faisabilité d'une imagerie de tissus profonds, une injection dans le muscle tibial cranial d'une souris Swiss a été effectuée avec une quantité supérieure de nanoparticules (20 µL à une concentration de 10 mg/mL de nanoparticules). Le signal était clairement détectable et définissait le contour du muscle de la souris. Alors que l'injection réalisée était ponctuelle, le signal observé était diffus dans l'ensemble du muscle. En supprimant le besoin d'illumination in situ, l'utilisation de nanoparticules à luminescence persistante permet ainsi d'observer de façon simple la diffusion de la lumière dans les tissus. Un signal provenant de la patte de l'animal a également pu être observé, alors qu'aucune nanoparticule n'y avait été injectée. Ceci a été attribué à la réflexion sur la patte de la souris du signal provenant du muscle.

Afin d'assurer le succès et d'élargir le champ d'application, les sondes optiques doivent pouvoir être fonctionnalisées. Des techniques d'enrobage classiquement utilisées pour les silicates ont été utilisées (Fig. 16). Après traitement thermique, des groupements hydroxyles sont créés à la surface des nanoparticules par érosion partielle de la surface par une base (NaOH, 5mM). Ces hydroxyles donnent à la nanoparticules un potentiel Zeta négatif à pH neutre (-34,3 mV) et permettent le greffage covalent de différents groupements fonctionnels. Les nanoparticules sont ensuite dispersées dans du diméthylformamide pour réagir avec 3-aminopropyl-triethoxysilane (APTES) qui va se greffer covalemment à la surface des nanoparticules. Il en résulte des nanoparticules chargées positivement à cause de la présence de groupements amine libre à la surface des nanoparticules. Ces particules seront nommées amino-NPs. Le succès du greffage a été suivi par mesure du potentiel Zéta des amino-NPs (+35.8 mV à pH~7) et par un test positif au trinitrobenzene sulfonate (TNBS). L'excès d'APTES a été enlevé par des étapes successives de sédimentation-lavage.

La charge de surface des amino-NPs a été inversée par réaction avec de l'anhydride diglycolique, qui réagit avec les amines libres. Cette réaction permet ainsi d'obtenir des groupements carboxyles à la surface des nanoparticules. Le potentiel Zéta de ces nanoparticules (dénommées carboxy-NPs) était comme attendu négatif à pH neutre (-37.3mV). Un couplage peptidique utilisant du $mPEG_{5000}$-COOH ($\alpha$-carboxy-$\omega$-methoxy-polyethyleneglycol F.W.:~5000g/mol) a été effectué sur les amines des amino-NPs. Ceci permet d'obtenir des particules neutres (potentiel Zéta de +5.1 mV à pH neutre) seront dénommées PEG-NPs. A chaque fois, l'ensemble des produits qui n'avaient pas réagi a été enlevé par des étapes de sédimentation-lavage. Trois types de nanoparticules portant des charges de surface différentes ont ainsi été obtenus.

Même s'il est connu que la charge de surface influence la distribution *in vivo* de particules, le suivi de biodistribution ne se fait généralement pas en temps réel. Par exemple, les animaux doivent être sacrifiés à des temps différents pour déterminer la cinétique de biodistribution de liposomes ou de nanoparticules. La biodistribution des différents types de nanoparticules à luminescence persistante a pu être quant à elle suivie par imagerie optique en temps réel chez la souris après injection dans la veine de la queue d'1 mg de nanoparticules (correspondant à $10^{13}$ nanoparticules).

Pour les amino-NPs chargés positivement, une rétention importante a pu être observée au niveau des poumons. Pendant la première heure, la biodistribution des nanoparticules a peu changé, avec simplement un transfert progressif mais lent des nanoparticules des poumons vers le foie et la rate. Deux raisons peuvent expliquer cette tendance de séquestration dans les poumons. La première est l'interaction électrostatique non spécifique des nanoparticules avec les protéines négatives couvrant la surface des cellules endothéliales, comme par exemple les glycosaminoglycanes. En effet, les poumons sont le premier organe fortement vascularisé que rencontrent les nanoparticules après une injection systémique dans la veine de la queue d'une souris. Le flux sanguin étant plus faible à cause de la circulation dans les capillaires, les interactions non spécifiques deviennent importantes par rapport aux autres interactions et ainsi bloquent les amino-NPs au niveau des poumons. Une autre explication peut provenir de l'aggrégation des nanoparticules avec les composés chargés négativement du sang empêchant ainsi la bonne circulation au niveau des capillaires des poumons et ainsi provoquant leur séquestration.

Pour les carboxy-NPs, chargées négativement, aucune séquestration pulmonaire n'a pu être observée. Ceci est probablement dû au fait que les nanoparticules négatives n'interagissent pas avec les cellules endothéliales des poumons et ainsi restent dans la circulation sanguine plus longtemps que les particules positives. Toutefois, une forte captation hépatique a pu être observée. Ceci résulte probablement d'une opsonisation et une captation des nanoparticules par les cellules endothéliales et de Kupffer du système réticuloendothélial (RES).

Il a été démontré par plusieurs auteurs que l'utilisation de PEG couvant la surface de nanoparticules permettait de réduire l'élimination rapide de la circulation sanguine. Ainsi les PEG-NPs ont confirmé cette tendance puisque le signal obtenu après injection était diffus et couvrait l'ensemble du corps de la souris, et ce durant la totalité de l'expérience (45 min). Toutefois, après 30 min, une accumulation au niveau du système réticuloendothéliale a pu être observée.

La capture de composés exogènes par le foie ou la rate est un problème évident qu'il est nécessaire de résoudre pour cibler d'autres tissus, comme les tumeurs. Certains auteurs ont décrit des techniques permettant d'éliminer ou de minimiser la capture par le RES. Parmi ces techniques, l'utilisation de liposomes anioniques contenant une quantité équimolaire de phosphatidylcholine, cholestérol et phosphatidylsérine permet de saturer les RES et ainsi de prolonger la durée de vie dans la circulation sanguine des composés injectés. Les liposomes ont été préparés par hydratation d'un film de lipides puis extrudés afin d'obtenir des liposomes négatifs (potentiel Zéta -43.3 mV) avec un diamètre d'environ 300 nm.

En diminuant le nombre de sites potentiels d'interaction, une préinjection intraveineuse de liposomes anioniques (6 $\mu$mol, 100 $\mu$L injecté 5 min avant l'injection de nanoparticules) a permis d'augmenter grandement le temps de circulation des carboxy-NPs chargés négativement chez la souris, même si ces particules se sont finalement localisées dans le foie et la rate.

L'effet d'une préinjection de liposomes anioniques a eu un effet encore plus important sur le temps de circulation des PEG-NPs. Le signal obtenu était en effet diffus tout au long de l'expérience montrant ainsi la présence des nanoparticules dans la vasculature de la souris. Il est à noter que les artères fémorales et les carotides ont pu être visualisées. Après 15 min, le contour de la rate était clairement visible alors que 30 min ont été nécessaires pour que le contour de foie ressorte de la circulation périphérique du haut du corps de la souris.

Ainsi, même si la préinjection de composés interagissant avec le RES n'altère pas la biodistribution finale des nanoparticules, cela améliore de façon nette le temps de circulation des nanoparticules et ainsi la possibilité d'obtenir un ciblage spécifique des nanoparticules.

Enfin, la forte vascularisation d'une tumeur a été mise en évidence grâce à l'utilisation des nanoparticules à luminescence persistante. Une tumeur de carcinome pulmonaire de Lewis (3LL) a été implantée dans la région inguinale à une souris C57B1/6. La souris préalablement rasée a été injecté avec des PEG-NPs (1 mg) 5 min après injection de liposomes anioniques (6 $\mu$mol, 100 $\mu$L). La présence de mélanine dans la peau des souris C57B1/6 est particulièrement désavantageuse pour réaliser des expériences en imagerie optique *in vivo*. En effet, le coefficient d'atténuation de la mélanine est très importante et couvre l'ensemble du spectre visible. Ainsi, l'intensité totale détecté était significativement inférieure (d'un rapport 5 à 7) en comparaison de celle obtenue pour une souris Swiss (Fig. 17). Toutefois, l'utilisation de nanoparticules à luminescence persistante a permis d'obtenir un signal facilement détectable et la biodistribution des nanoparticules a été suivie par mesure optique. La région tumorale a ainsi pu être mis en évidence par une augmentation de l'intensité lumineuse à cet endroit. Cette augmentation est attribuée à la forte vascularisation de tumeur 3LL.

**Synthèse des nanoparticules de composition $Ca_{0.2}Zn_{0.9}Mg_{0.9}Si_2O_6$ dopés $Eu^{2+}$ (0,5%), $Dy^{3+}$(1,0%), $Mn^{2+}$ (2,5%)**

Les produits chimiques utilisés pour synthétiser les nanoparticules sont le nitrate de magnesium ($Mg(NO_3)_2,6H_2O$), le chlorure de zinc ($ZnCl_2$), le chlorure de calcium ($CaCl_2,2H_2O$), le chlorure de europium ($EuCl_3,6H_2O$), le nitrate de dysprosium ($Dy(NO_3)_3,5H_2O$), le chlorure de manganese ($MnCl_2,4H_2O$) et le tetraethoxysilane (TEOS). Tous les sels sont dissous dans de l'eau acidifiée à pH2 par addition d'acide nitrique concentré. Le TEOS est alors ajouté rapidement et la solution est agitée vigoureusement à température ambiante jusqu'à l'obtention d'une solution limpide (environ 1h). La solution est alors chauffée à 70°C jusqu'à gélification (environ 2h). Le gel mouillé est alors séché dans une étuve à 110°C pendant 20h. Le gel opaque obtenu est directement calciné dans un creuset en zircone sous atmosphère réductrice (Noxal 4 : 10% $H_2$,

90% Ar) à 1050°C pendant 10h. La matériau obtenu est alors broyé dans un mortier pour obtenir les nanoparticules désirées.

La poudre est dispersée par sonication dans une solution de soude (5mM) à une concentration de 10 mg de nanoparticules par mL. Après neutralisation avec une solution d'acide chlorhydrique, la suspension est diluée avec de l'eau distillée jusqu'à obtenir une suspension à une concentration de 2,5 mg de nanoparticules par mL. Cette suspension est alors centrifugée à l'aide d'une centrifugeuse SANYO MSE Mistral 1000 (2000 rpm pendant 15min) afin d'éliminer les plus grosses particules. Au surnageant est ajouté 25% en volume d'acétone. Ceci permet de faciliter la sédimentation des nanoparticules lors de la centrifugation à 4500rpm pendant 30 min qui permet de récupérer les plus petites particules. Après avoir enlevé le surnageant, le culot constitué des nanoparticules est récupéré. Les nanoparticules sont ensuite séchées dans une étuve sous vide.

**Autre méthode de modification de surface**

*Exemple de fonctionnalisation pour le composé $Ca_{0.2}Zn_{0.9}Mg_{0.9}Si_2O_6$ dopés $Eu^{2+}$ (0,5%),$Dy^{3+}$(1,0%), $Mn^{2+}$ (2,5%)*

Pour obtenir les amino-NPs, 100 mg de nanoparticules sont dispersées dans 10 mL de dimethylformamide (DMF) et 50 $\mu$L de 3-aminopropyl-triethoxysilane (APTES) sont ajoutés sous agitation. La suspension est alors agitée pendant une nuit à 80°C. Après réaction, plusieurs séries de centrifugations et redispersions dans le DMF permettent de laver l'excès d'APTES.

Pour obtenir les carboxy-NPs, 52.8mg d'amino-NPs sont redispersées dans du DMF et de l'anhydride diglycolique (12.2 mg, 0.11 mmol) est ajouté. La suspension est alors agitée pendant une nuit à température ambiante. Après réaction, l'excès de réactifs est enlevé par des lavages successifs.

Pour obtenir les PEG-NPs, 53.4 mg d'amino-NPs sont redispersées dans 10 mL de DMF en présence de Methoxy-$PEG_{5000}$-COOH (534 mg, 0.11 mmol), benzotriazol-1-yl-oxy-tris(dimethylamino)-phosphonium hexafluorophosphate (BOP reagent, 52 mg, 0.12 mmol) and thriethylamine (15 $\mu$L). L'excès de réactifs est enlevé par des lavages successifs.

**Synthèse de liposomes anioniques**

De la Phosphatidylcholine (11.78 mg, 15.5 $\mu$mol), du cholesterol (5.99 mg, 15.5 $\mu$mol) et de la L -phosphatidylserine (12.2 mg, 15.5 $\mu$mol) sont dissouts dans 1mL de chloroforme. Après élimination du solvant par évaporation sous vide, le film lipidique obtenu est hydrate à 37°C toute la nuit avec 1.935 mL de PBS 10 mM. La suspension est alors extrudée à travers des filtres de 0,4 $\mu$m à l'aide d'un mini-extruder (Avanti Polar Lipids). Avant injection, la suspension de liposomes est diluée avec du PBS 10 mM afin d'obtenir une suspension de liposomes anioniques à une concentration en lipide de 6 mM.

**Analyse des données pour une injection sous-cutanée.**

Pour la dose la plus faible (20 ng), l'intensité du signal était supérieure à 10 alors que le signal du bruit de fond a une intensité moyenne de 2,5 avec une variance de 1,5. Le rapport signal sur bruit est calculé comme le rapport entre l'amplitude du signal (∼7,5) et la variance du bruit (1,5) est donc supérieur à 5 (voir la Fig. 18).

**Evaluation qualitative du temps de circulation des nanoparticules**

Pour évaluer le temps de circulation des nanoparticules (NPs), une région d'intérêt (ROI) couvrant les poumons, le foie et la région de la rate (ROI_1) a été sélectionnée manuellement et analysée par période de 20 s. L'intensité a ensuite été divisée par l'intensité lumineuse totale du corps (ROI_2) détectée pour chaque souris.

Ainsi, la concentration sanguine des nanoparticules est liée à la fonction

$$F(t) = 1 - ROI\_1/ROI\_2.$$

Comme décrit dans la Figure 19, pour les amino-NPs, le pourcentage de luminescence dans la région RES-poumons est élevé (75%) une seconde après l'injection et ne fluctue pratiquement pas. Pour les carboxy-NPs, la courbe diminue rapidement de 0,5 à 0,2 en approximativement 10 min, et la pré-injection de liposomes anioniques augmente le niveau initial à 0,65. Le temps de circulation est également prolongé au vu du temps nécessaire pour retrouver des niveaux similaires au cas où les NPs sont injectées seules. Pour les PEG-NPs, le niveau initial est élevé (0,65). La courbe diminue plus lentement, et le niveau est finalement de 0,4 après 1 heure. Ce niveau est plus faible que pour l'autre type de NPs résultant de « l'effet stealth » du domaine PEG sur le temps de circulation. La pré-injection de liposome avant l'injection PEG-NPs permet une circulation des NPs durable.

**Expériences de suivi à long terme :**

L'expérience suivante a été réalisée pour savoir si les nanoparticules pouvaient être éliminées à plus long terme.

Un groupe de 30 souris Swiss a reçu le même jour une injection i.v. avec une suspension de nanoparticules non fonctionnalisées (1 mg par souris). Le groupe contrôle a été injecté avec du sérum physiologique.

Aucune souris n'est morte lors de l'injection. Une légère baisse de poids du groupe injecté par rapport au groupe témoin a été observée durant les premiers jours. Toutefois, au temps plus long, les deux groupes étaient d'un poids similaire (Fig. 20). Ceci tendrait à montrer que les nanoparticules n'ont pas d'effet de toxicité aiguë sur la souris. Des études plus précises avec des injections répétées devraient être effectuées pour mieux appréhender la toxicité des nanoparticules. Pour savoir si les nanoparticules pouvaient être éliminées, les souris ont été sacrifiées par groupe de 4, leur organes ont été prélevées (foie, rate, reins et poumons) et la quantité de nanoparticules dosée dans chaque organe.

Les résultats de cette analyse ont montré que la quantité de nanoparticules était, comme attendu, largement plus importante dans le foie que dans les autres organes (cf Fig. 21-24). Les Inventeurs ont observé au cours des premiers jours une augmentation du nombre de nanoparticules dans le foie, montrant le mécanisme de capture par le foie. Au temps long, la tendance s'est inversée avec de moins en moins de particules présentes dans l'ensemble des organes. Au temps M6 (6 mois après l'injection), la quantité de nanoparticules retrouvées dans les organes était quasiment nulle.

Les Inventeurs estiment que l'élimination des particules peut se faire par la voie biliaire.

**Visualisation de tumeurs chez la souris :**

Les Inventeurs ont voulu savoir si les nanoparticules étaient capables de révéler l'emplacement d'une tumeur implantée chez la souris. Deux types de tumeurs ont été utilisées : une tumeur issu d'un carcinome pulmonaire de Lewis (3LL) et une tumeur de type mélanome B16.

Une des particularités des tumeurs 3LL est d'être fortement vascularisée. On observe ainsi une augmentation du signal au niveau de la tumeur, ce qui permet de distinguer clairement le contour de la tumeur. Pour ce qui est des tumeurs B16, issu de mélanome, elles contiennent une importante quantité de mélanine, pigment absorbant fortement la lumière. Ainsi, la tumeur a été dans ce cas révélée par une absence de signal provenant de la tumeur.

**Expériences sur le rat :**

Les Inventeurs ont voulu savoir si la lumière émise par les nanoparticules était suffisante pour faire une imagerie sur des animaux de taille plus importante qu'une souris.

Ils ont donc tester si un suivi des nanoparticules lors d'une injection systémique sur un rat wistar (300 g) pouvait être réalisé. Pour comparaison, le poids d'une souris varie de 20 à 30 g.

Une quantité de particules équivalente à celles utilisées pour les souris (1 mg) a donc été injectée en intraveineuse par la veine de la queue. Une captation hépatique rapide a ainsi pu être mise en évidence sur le rat.

**Revendications**

1. Nanoparticule à luminescence persistante pour son utilisation in vivo en tant qu'agent de diagnostic destiné à l'imagerie optique, ladite nanoparticule émettant des photons à des longueurs d'onde comprises entre 400 et 1300 nm pendant au moins 0,01 seconde, après une excitation lumineuse à des longueurs d'onde comprises entre 100 et 800 nm, ou encore après excitation par des rayons X.

2. Nanoparticule pour son utilisation selon la revendication 1, **caractérisée en ce qu'**elle a une taille comprise entre 25 nm et 1 $\mu$m.

3. Nanoparticule pour son utilisation selon la revendication 1 ou 2, **caractérisée en ce qu'**elle est constituée par un composé choisi dans le groupe constitué par :

    - les silicates, les aluminates, les aluminosilicates, les germanates, les titanates, les oxysulfures, les phosphates et les vanadates, de tels composés comprenant au moins un oxyde de métal,
    - les sulfures comprenant au moins un ion métallique choisi parmi le zinc, le strontium et le calcium, et
    - les oxydes de métaux,

    ledit composé étant dopé avec au moins un ion terre rare, et éventuellement avec au moins un ion d'un métal de transition.

4. Nanoparticule pour son utilisation selon la revendication 3, **caractérisée en ce que** le métal de l'oxyde de métal est choisi parmi le magnésium, le calcium, le strontium, le baryum, le zinc, le cadmium, l'yttrium et le gallium.

5. Nanoparticule pour son utilisation selon la revendication 3 ou 4, **caractérisée en ce que** l'ion terre rare est choisi parmi les ions europium, ytterbium, cérium, samarium, praséodyme, dysprosium, néodyme, holmium, terbium, thulium et erbium.

6. Nanoparticule pour son utilisation selon l'une des revendications 3 à 5, **caractérisée en ce que** le métal de transition est choisi parmi le manganèse, le chrome et le titane.

7. Nanoparticule pour son utilisation selon l'une des revendications 3 à 6, **caractérisée en ce qu'**elle est constituée par un silicate comprenant un oxyde de métal dopé avec au moins un ion terre rare et au moins un ion d'un métal de transition.

8. Nanoparticule pour son utilisation selon la revendication 7, **caractérisée en ce qu'**elle est constituée par un composé choisi dans le groupe constitué par les silicates ZnMgSi206, CaMgSi$_2$O$_6$ et MgSiO$_3$, de tels silicates étant dopés avec les ions manganèse, europium et dysprosium, et Sr2MgSÎ207 dopé avec les ions europium et dysprosium.

9. Nanoparticule pour son utilisation selon la revendication 7, **caractérisée en ce qu'**elle est constituée par le silicate Ca0.2Zn0.9Mg0.9Si2O6 dopé Eu2+, Ly3+, Mn2.

10. Nanoparticule pour son utilisation selon l'une des revendications 1 à 9, **caractérisée en ce qu'**elle est fonctionnalisée par enrobage et greffage de ligand permettant une liaison avec une substance d'intérêt biologique ou chimique.

11. Nanoparticule pour son utilisation selon l'une quelconque des revendications 1 à 10, **caractérisée en ce qu'**elle est excitée avant l'administration.

12. Nanoparticule pour son utilisation selon l'une quelconque des revendications 1 à 11, **caractérisée en ce que** l'agent de diagnostic est destiné à l'imagerie de la vascularisation de l'organisme.

13. Nanoparticule pour son utilisation selon l'une quelconque des revendications 1 à 12, **caractérisée en ce que** l'agent de diagnostic est destiné à l'imagerie de zones tumorales, inflammatoires, de la rétine, lesdites zones étant susceptibles d'être hypervascularisées, ou encore des zones de rupture de la barrière hémato- encéphalique.

14. Nanoparticule pour son utilisation selon l'une quelconque des revendications 1 à 12, **caractérisée en ce que** l'agent de diagnostic est destiné à l'imagerie de zones hypovascularisées telles que dans le cas d'une ischémie cérébrale ou cardiaque, ou dans le cas d'un trauma crânien.

**Patentansprüche**

1. Nanopartikel mit anhaltender Lumineszenz für dessen Verwendung in vivo als Diagnosemittel, das zur optischen Abbildung vorgesehen ist, wobei das Nanopartikel nach einer Lichtanregung mit Wellenlängen zwischen 100 und 800 nm oder auch nach einer Anregung durch Röntgenstrahlen Photonen mit Wellenlängen zwischen 400 und 1300 nm für mindestens 0,01 Sekunden emittiert.

2. Nanopartikel für dessen Verwendung nach Anspruch 1, **dadurch gekennzeichnet, dass** es eine Größe zwischen 25 nm und 1 $\mu$m aufweist.

3. Nanopartikel für dessen Verwendung nach Anspruch 1 oder 2, **dadurch gekennzeichnet, dass** es aus einer Verbindung besteht, die aus der Gruppe ausgewählt ist, die aus folgenden besteht:

   - Silicaten, Aluminaten, Alumosilicaten, Germanaten, Titanaten, Oxidsulfiden, Phosphaten und Vanadaten, wobei derartige Verbindungen mindestens ein Metalloxid umfassen,
   - Sulfiden, die mindestens ein Metallion umfassen, das aus Zink, Strontium und Calcium ausgewählt ist, und
   - Metalloxiden,

   wobei die Verbindung mit mindestens einem Seltenerdmetallion und gegebenenfalls mit mindestens einem Übergangsmetallion dotiert ist.

4. Nanopartikel für dessen Verwendung nach Anspruch 3, **dadurch gekennzeichnet, dass** das Metall des Metalloxids aus Magnesium, Calcium, Strontium, Barium, Zink, Cadmium, Yttrium und Gallium ausgewählt ist.

5. Nanopartikel für dessen Verwendung nach Anspruch 3 oder 4, **dadurch gekennzeichnet, dass** das Seltenerdmetallion aus Europium-, Ytterbium-, Cer-, Samarium-, Praseodym-, Dysprosium-, Neodym-, Holmium-, Terbium-, Thulium- und Erbiumionen ausgewählt ist.

6. Nanopartikel für dessen Verwendung nach einem der Ansprüche 3 bis 5, **dadurch gekennzeichnet, dass** das Übergangsmetall aus Mangan, Chrom und Titan ausgewählt ist.

7. Nanopartikel für dessen Verwendung nach einem der Ansprüche 3 bis 6, **dadurch gekennzeichnet, dass** es aus einem Silicat besteht, das ein Metalloxid umfasst und mit mindestens einem Seltenerdmetallion und mindestens einem Übergangsmetallion dotiert ist.

8. Nanopartikel für dessen Verwendung nach Anspruch 7, **dadurch gekennzeichnet, dass** es aus einer Verbindung besteht, die aus der Gruppe bestehend aus den Silicaten $ZnMgSi_2O_6$, $CaMgSi_2O_6$ und $MgSiO_3$, wobei derartige Silicate mit Mangan-, Europium- und Dysprosiumionen dotiert sind, und $Sr_2MgSi_2O_7$, das mit Europium- und Dysprosiumionen dotiert ist, ausgewählt ist.

9. Nanopartikel für dessen Verwendung nach Anspruch 7, **dadurch gekennzeichnet, dass** es aus dem Silicat $Ca_{0,2}Zn_{0,9}Mg_{0,9}Si_2O_6$ besteht, das mit $Eu^{2+}$, $Ly^{3+}$, $Mn^2$ dotiert ist.

10. Nanopartikel für dessen Verwendung nach einem der Ansprüche 1 bis 9, **dadurch gekennzeichnet, dass** es durch Beschichten und Pfropfen eines Liganden funktionalisiert wird, was eine Verbindung mit einer biologischen oder chemischen Substanz von Interesse ermöglicht.

11. Nanopartikel für dessen Verwendung nach einem der Ansprüche 1 bis 10, **dadurch gekennzeichnet, dass** es vor der Verabreichung angeregt wird.

12. Nanopartikel für dessen Verwendung nach einem der Ansprüche 1 bis 11, **dadurch gekennzeichnet, dass** das Diagnosemittel zur Abbildung der Vaskularisierung eines Organismus vorgesehen ist.

13. Nanopartikel für dessen Verwendung nach einem der Ansprüche 1 bis 12, **dadurch gekennzeichnet, dass** das Diagnosemittel zur Abbildung von Tumorbereichen, Entzündungsbereichen, Bereichen der Retina, wobei diese Bereiche für eine Hypervaskularisierung anfällig sind, oder auch Bereiche einer Ruptur der Blut-Hirn-Schranke vorgesehen ist.

14. Nanopartikel für dessen Verwendung nach einem der Ansprüche 1 bis 12, **dadurch gekennzeichnet, dass** das Diagnosemittel zur Abbildung von hypovaskularisierten Bereichen, wie im Fall einer zerebralen oder kardialen Ischämie oder im Fall eines Schädeltraumas, vorgesehen ist.

## Claims

1. Persistent luminescence nanoparticle for use thereof *in vivo* as a diagnosis agent, said nanoparticle emitting photons at wavelengths between 400 and 1300 nm for at least 0.01 seconds, after light excitation at wavelengths between 100 and 800 nm, or after excitation by means of X-rays.

2. Nanoparticle for use thereof according to claim 1, **characterized in that** it is between 25 nm and 1 $\mu$m in size.

3. Nanoparticle for use thereof according to claim 1 or 2, **characterized in that** it consists of a compound selected from the group consisting of:

   - silicates, aluminates, aluminosilicates, germanates, titanates, oxysulfides, phosphates and vanadates, such compounds comprising at least one metal oxide,
   - sulfides comprising at least one metal ion selected from zinc, strontium and calcium, and

- metal oxides,

said compound being doped with at least one rare earth ion, and possibly with at least one transition metal ion.

4. Nanoparticle for use thereof according to claim 3, **characterized in that** the metal of the metal oxide is selected from magnesium, calcium, strontium, barium, zinc, cadmium, yttrium and gallium.

5. Nanoparticle for use thereof according to claim 3 or 4, **characterized in that** the rare earth ion is selected from europium, ytterbium, cerium, samarium, praseodymium, dysprosium, neodymium, holmium, terbium, thulium and erbium ions.

6. Nanoparticle for use thereof according to one of claims 3 to 5, **characterized in that** the transition metal is selected from manganese, chromium and titanium.

7. Nanoparticle for use thereof according to one of claims 3 to 6, **characterized in that** it consists of a silicate comprising a metal oxide doped with at least one rare earth ion and at least one transition metal ion.

8. Nanoparticle for use thereof according to claim 7, **characterized in that** it consists of a compound selected from the group consisting of the silicates $ZnMgSi_2O_6$, $CaMgSi_2O_6$ and $MgSiO_3$, such silicates being doped with manganese, europium and dysprosium ions, and $Sr_2MgSi_2O_7$ doped with europium and dysprosium ions.

9. Nanoparticle for use thereof according to claim 7, **characterized in that** it consists of the silicate $Ca_{0.2}Zn_{0.9}Mg_{0.9}Si_2O_6$, doped with $Eu^{2+}$, $Ly^{3+}$ and $Mn2^+$.

10. Nanoparticle for use thereof according to one of claims 1 to 9, **characterized in that** it is functionalized by coating and grafting a ligand enabling bonding with a substance of biological or chemical interest.

11. Nanoparticle for use thereof according to any one of claims 1 to 10, **characterized in that** it is excited before administration.

12. Nanoparticle for use thereof according to any one of claims 1 to 11, **characterized in that** the diagnosis agent is intended for imaging of the vascularisation of the body.

13. Nanoparticle for use thereof according to any one of claims 1 to 12, **characterized in that** the diagnostic agent is intended for imaging tumoral area, inflammatory area, retina area, said areas being possibly hypervascular area, or also area of breach of the hemato-encephalo barrier.

14. Nanoparticle for use thereof according to any one of claims 1 to 12, **characterized in that** the diagnostic agent is intended for imaging hypervascular area such as in case of brain or cardiac ischemia or in case of head trauma.

**Figure 1A**

**Figure 1B**

**Figure 2**

**Figure 3**

**Figure 4**

**Figure 5**

**Figure 6**

**Figure 7**

**Figure 8**

**Figure 9**

**Figure 10**

**Figure 11**

**Figure 12**

● : électron
□ : trou
exciton : paire électron-trou

**Figure 13**

14A

14B

14C

14D

14E

Figure 14

Detecteur

hν

Figure 15

**Figure 16**

**Figure 17**

**Figure 18**

—△— amino-NPs
—○— carboxy-NPs
—◇— PEG-NPs
······· carboxy-NPs avec préinjection de liposomes anioniques
—▽— PEG-NPs avec préinjection de liposomes anioniques

**Figure 19**

Figure 20

Figure 21

## Poumons

**Figure 22**

## Rate

**Figure 23**

**Reins**

Figure 24

## RÉFÉRENCES CITÉES DANS LA DESCRIPTION

**Documents brevets cités dans la description**

- US 6537829 B
- US 2003059635 A
- US 2004014060 A
- US 2003180780 A

**Littérature non-brevet citée dans la description**

- **JIANG et al.** *Journal of Alloys and Compounds,* 2004, vol. 377, 211-215
- **G. BLASSE ; B.C. GRABMAIER.** Luminescent materials. Springer-Verlag, 1994, 91
- *Applied Optics,* 1973, vol. 12, 555-563